(19) Europäisches Patentamt
European Patent Office
Office européen des brevets

(11) **EP 4 768 580 A2**

## (12) EUROPEAN PATENT APPLICATION

(43) Date of publication:
**01.07.2026 Bulletin 2026/27**

(21) Application number: 26163371.3

(22) Date of filing: **19.01.2024**

(51) International Patent Classification (IPC):
***C12N 15/10*** *(2006.01)*

(52) Cooperative Patent Classification (CPC):
**C07K 14/33; A61K 38/4893; C12Y 304/24069;**
**Y02A 50/30**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **20.01.2023 US 202363480938 P**

(62) Document number(s) of the earlier application(s) in
accordance with Art. 76 EPC:
**24745256.8 / 4 652 187**

(71) Applicant: **AbbVie Inc.**
**North Chicago, IL 60064 (US)**

(72) Inventors:
• **KEHRER, Robert, R.**
**North Chicago, IL 60064 (US)**
• **WIIG, Jared**
**Fullerton, CA 92831 (US)**
• **XIANG, Hui**
**Irvine, CA 92618 (US)**
• **NGUYEN, Phillip, P.**
**Irvine, CA 92618 (US)**

• **PATEL, Hemant, A.**
**Santa Margarita, CA 92688 (US)**
• **NG, Connie, J.**
**Irvine, CA 92614 (US)**
• **GUERRERO, Cortnie, M.**
**Costa Mesa, CA 92626 (US)**

(74) Representative: **Jones Day**
**Gewürzmühlstr. 11**
**80538 München (DE)**

Remarks:
•The complete document including Reference
Table(s) and the Sequence Listing(s) can be
downloaded from the EPO website
•This application was filed on 09-03-2026 as a
divisional application to the application mentioned
under INID code 62.
•Claims filed after the date of filing of the application/
after the date of receipt of the divisional application
(Rule 68(4) EPC).

### (54) CLOSTRIDIUM BOTULINUM NEUROTOXIN SEROTYPE A COMPOSITIONS

(57) The present disclosure relates to methods of producing a 900 kDa BoNT/A complex, wherein the method does not comprise a step of precipitation during the purification process. Further provided herein are methods of preparing a *Clostridium botulinum* working cell bank (WCB).

EP 4 768 580 A2

**Description**

**CROSS-REFERENCE TO RELATED APPLICATIONS**

This application claims the benefit of U.S. Provisional Application No. 63/480,938, filed on January 20, 2023, which is incorporated by reference herein in its entirety.

**REFERENCE TO SEQUENCE LISTING SUBMITTED ELECTRONICALLY**

This application incorporates by reference a Sequence Listing submitted with this application as an xml file entitled "13371-270-228_SEQ_LISTING.xml" created on January 11, 2024 and having a size of 6,707 bytes.

**1. FIELD**

Provided herein are compositions comprising *Clostridium botulinum* neurotoxin serotype A (BoNT/A) that have a low level of oxidation at specific methionine positions of 150 kDa BoNT/A. Further provided herein are methods of producing a composition comprising BoNT/A using a low temperature during the process of fermenting *Clostridium botulinum* bacteria.

**2. BACKGROUND**

*Clostridium botulinum* neurotoxin serotype A (BoNT/A) is a highly potent toxin that causes muscle relaxation by inhibition of synaptic vesicle docking and fusion, thereby blocking acetylcholine release at neuromuscular junctions. BoNT/A is produced by *Clostridium botulinum* Type A strains, which synthesize a complex of a 150 kDa neurotoxin, along with a group of nontoxic neurotoxin-associated proteins (NAPs). BOTOX®, or onabotulinumtoxinA, is a BoNT/A product approved by the United States Food and Drug Administration (FDA) in 1989 for a variety of therapeutic and cosmetic indications.
To date, there remains a need for developing *botulinum* toxins pharmaceutical compositions with improved properties.
Citation of a reference herein shall not be construed as an admission that such is prior art to the present disclosure.

**3. SUMMARY**

The present disclosure is based, in part, on the surprising finding that modulating the temperature during the process of fermenting *Clostridium botulinum* produced compositions with varied levels of oxidized methionine residues of the produced *Clostridium botulinum* neurotoxin serotype A (BoNT/A). For example, using a temperature that is lower than the standard fermentation temperature either for a short period of time (*i.e.,* cold shock) or continuously during the fermentation *of Clostridium botulinum* in animal protein free cell culture media reduced the oxidation level at one or more of the methionine residues (*e.g.*, M411, M550, M1004, and/or M1144) of the produced 150 kDa neurotoxin component of BoNT/A. The present disclosure also identifies a negative correlation between the oxidation level and the potency of BoNT/A-that is, BoNT/A with lower oxidation level exhibits higher potency.
Accordingly, in one aspect, the present disclosure relates to a composition comprising a plurality of 900 kDa *Clostridium botulinum* serotype A (BoNT/A) neurotoxin complex species, wherein the oxidation level of 150 kDa neurotoxin species present in the composition is less than 6% at each of the following positions: 411 (M411) as shown in SEQ ID NO:2, 550 (M550) as show in SEQ ID NO:3, 1004 (M1004) as shown in SEQ ID NO:3, and 1144 (M1144) as shown in SEQ ID NO:3.
In an embodiment, the 900 kDa BoNT/A complex is onabotulinumtoxinA.
In one embodiment, the oxidation level at each said position is less than 6%.
In certain embodiments, the oxidation level at position M411 of SEQ ID NO. 2 is less than 5%. In certain embodiments, the oxidation level at position M411 of SEQ ID NO. 2 is about 2%.
In certain embodiments, the oxidation level at position M550 of SEQ ID NO. 3 is less than 1%. In certain embodiments, the oxidation level at position M550 of SEQ ID NO. 3 is about 0.3% to about 0.5%.
In certain embodiments, the oxidation level at position M1004 of SEQ ID NO. 3 is less than 3%. In certain embodiments, the oxidation level at position M1004 of SEQ ID NO. 3 is about 1% to about 2%.
In certain embodiments, the oxidation level at position M1144 of SEQ ID NO. 3 is less than 6%. In certain embodiments, the oxidation level at position M1144 of SEQ ID NO. 3 is about 2%.
In certain embodiments, the oxidation level is determined by LC-MS/MS.

In certain embodiments, the composition comprises less than 3% of host cell protein.

In certain embodiments, the composition has a potency of about $1.5 \times 10^7$ units/mg to about $6.0 \times 10^7$ units/mg. In certain embodiments, the potency is determined using a mouse 50% lethal dose (MLD50) assay. In certain embodiments, the potency is determined using a cell based potency assay.

In certain embodiments, the composition further comprises a pharmaceutically acceptable carrier. In certain embodiments, the pharmaceutically acceptable carrier comprises human serum albumin and sodium chloride. In certain embodiments, the composition is vacuum-dried.

In certain embodiments, the 900 kDa BoNT/A complex is produced in a fermentation condition comprising a cold shock fermentation temperature.

In certain embodiments, the 900 kDa BoNT/A complex is produced in a continuous cold temperature fermentation condition.

In certain embodiments, the 900 kDa BoNT/A complex is purified by one or more steps of column chromatography. In certain embodiments, the 900 kDa BoNT/A complex is purified by one or more steps of column chromatography that comprise hydrophobic interaction chromatography. In certain embodiments, the one or more steps of column chromatography further comprise anion exchange chromatography. In certain embodiments, the one or more steps of column chromatography further comprise cation exchange chromatography.

In certain embodiments, the 900 kDa BoNT/A complex is produced by *Clostridium botulinum* bacteria cultured and expanded from an animal product free working cell bank.

In certain embodiments, the composition is animal product free.

In certain embodiments, the composition does not contain a protease inhibitor.

In certain embodiments, the composition does not contain benzamidine hydrochloride.

In another aspect, provided herein is a method of producing a composition comprising BoNT/A, said method comprising incubating a culture of *Clostridium botulinum* bacteria in a production fermentor at a temperature that is below 35 °C for a period of time.

In certain embodiments, the entire fermentation period is about 72 hours. In certain embodiments, the entire fermentation period is about 160 hours.

In certain embodiments, the period of time is about 5 hours. In certain embodiments, the period of time is the entire fermentation period.

In certain embodiments, the temperature is about 15 °C. In certain embodiments, the temperature is about 20 °C. In certain embodiments, the temperature is about 25 °C.

In certain embodiments, the method comprises, in the following order: (a) incubating the culture of *Clostridium botulinum* bacteria in the production fermentor at 35 °C for about 12 hours; (b) setting temperature to 15 °C at the end of step (a); (c) culturing at 15 °C until about 5 hours after step (a); (d) setting temperature to 35 °C at the end of step (c); and (e) culturing at 35 °C until about 55 hours after step (c).

In certain embodiments, the method comprises, in the following order: (a) incubating the culture of *Clostridium botulinum* bacteria in the production fermentor at 35 °C for about 12 hours; (b) setting temperature to 20 °C at the end of step (a); (c) culturing at 20 °C until about 5 hours after step (a); (d) setting temperature to 35 °C at the end of step (c); and (e) culturing at 35 °C until about 55 hours after step (c).

In certain embodiments, the method comprises incubating the culture of *Clostridium botulinum* bacteria in the production fermentor at 25 °C for about 160 hours.

In another aspect, the present disclosure also relates to a method of treating a patient in need thereof, comprising administering a composition described herein.

## 4. BRIEF DESCRIPTION OF THE DRAWINGS

**FIG. 1** shows relative potency of 150 kDa BoNT/A DS treated with up to 100 pm of $H_2O_2$.

**FIG. 2** shows percentage increase of oxidation at methionine residues of 150 kDa BoNT/A DS treated with up to 100 ppm of $H_2O_2$.

**FIG. 3** shows 150 kDa BoNT/A with predominant oxidation sites labeled.

**FIG. 4** shows that oxidation of the 150 kDa BoNT/A subunit correlated with potency loss.

## 5. DETAILED DESCRIPTION

The present disclosure provides new BoNT/A compositions with reduced oxidation levels and improved properties as well as methods for producing same. Accordingly, in one aspect, the present disclosure relates to a composition comprising a plurality of 900 kDa *Clostridium botulinum* serotype A (BoNT/A) neurotoxin complex species, wherein the oxidation level of 150 kDa neurotoxin species present in the composition is less than 6% at each of the following positions: 411 (M411) as shown in SEQ ID NO:2, 550 (M550) as show in SEQ ID NO:3, 1004 (M1004) as shown in SEQ ID NO:3, and 1144 (M1144) as shown in SEQ ID NO:3.

Further benefits of the present disclosure will be apparent to one skilled in the art from reading this patent application. The embodiments of the disclosure described in the following paragraphs are intended to illustrate the invention and should not be deemed to narrow the scope of the invention.

### 5.1. Definitions

The singular forms "a," "an" and "the" include plural referents unless the context clearly dictates otherwise.

The term "and/or" as used in a phrase such as "A and/or B" herein is intended to mean "A and B", "A or B", "A" or "B".

The terms "about" and "approximately" generally refer to a range of numbers that one of skill in the art would consider equivalent to the recited value (*i.e.,* having the same function or result). In many instances, the terms "about" and "approximately" may include numbers that are rounded to the nearest significant figure. In specific embodiments, the terms "about" and "approximately" shall be construed so as to allow normal variation as judged by a person of ordinary skill in the art, such as, for example, a variation within 20% or 10% or 5%. In specific embodiments, the terms "about" and "approximately" encompass the exact value recited.

"Animal product free" ("APF") or "substantially animal product free" encompasses, respectively, the absence or substantial absence of blood derived, blood pooled and other animal derived products or compounds. "Animal" excludes microorganisms, such as bacteria. Thus, an APF medium or process or a substantially APF medium or process within the scope of the present invention can include a *botulinum* toxin or a *Clostridial botulinum* bacterium. For example, an APF process or a substantially APF process means a process which is either substantially free or essentially free or entirely free of animal-derived proteins, such as immunoglobulins, meat digest, meat by-products and milk or dairy products or digests.

"*Clostridium botulinum* neurotoxin serotype A" or "BoNT/A" means a neurotoxin produced by *Clostridium botulinum* Type A strains. One such *Clostridium botulinum* Type A strain is the Type A-Hall strain, for example, the Type A-Hall (Allergan) strain. Zhang et al. (2003) Gene 315:21, incorporated herein by reference in its entirety. BoNT/A encompasses both a BoNT/A complex (*e.g.,* the 300, 500, 760, and 900 kDa complexes) as well as pure BoNT/A toxin (i.e. the about 150 kDa neurotoxic molecule). Unless specified otherwise, BoNT/A used herein include both oxidized and unoxidized forms.

"BoNT/A complexes" means *Clostridium botulinum* serotype A neurotoxin complexes comprising a BoNT/A molecule (the neurotoxic component) and one or more hemagglutinin (HA) proteins and/or non-toxin non-hemagglutinin (NTNH) protein. The BoNT/A complexes can be in the forms of, *e.g.,* about 900 kDa, 760 kDa, 500 kDa or 300 kDa. In one embodiment, the BoNT/A complex is in the form of about 900 kDa comprising an about 150 kDa BoNT/A molecule, hemagglutinin HA70, hemagglutinin HA34, hemagglutinin HA17, and nontoxic-nonhemagglutinin (NTNH) proteins. In one embodiment, the BoNT/A complex is a substantially complete form of the 900 kDa BoTN/A complex. In one embodiment, the BoNT/A complex is onabotulinumtoxinA.

"150 kDa *Clostridium botulinum* serotype A neurotoxin" or "150 kDa BoNT/A" means a neurotoxin of approximately 150 kDa made from a culture of *Clostridium botulinum* type A strain (*e.g.,* the Hall strain of *Clostridium botulinum*). The preferred sequences of 150 kDa *botulinum* toxin type A (BoNT/A) used in the context of the present disclosure are shown in Table 1. For example, in one embodiment, the 150 kDa BoNT/A used in the context of the present disclosure comprises (*e.g.,* consists of) a light chain having an amino acid sequence set forth in SEQ ID NO. 2 and a heavy chain having an amino acid sequence set forth in SEQ ID NO. 3, with disulfide bridges located between positions 429 and 453 and between positions 1234 and 1279. Unless specified otherwise, 150 kDa BoNT/A used herein include both oxidized and unoxidized forms.

"BoNT/A composition" refers to any composition comprising BoNT/A and encompasses both solid compositions and liquid compositions. In certain embodiments, a BoNT/A composition (*e.g.,* a solid composition or liquid composition) described herein is a pharmaceutical composition. In specific embodiments, a BoNT/A composition (*e.g.,* a solid composition or liquid composition) described herein is a drug product (*i.e.,* a finished dosage form). In specific embodiments, a BoNT/A composition (*e.g.,* a solid composition or liquid composition) described herein is a drug substance. In a specific embodiment, a BoNT/A composition described herein is in the form of a solution. In a specific embodiment, a BoNT/A composition described herein is in the form of powder (*e.g.,* vacuum-dried powder or freeze-dried powder).

"Cold shock" means a fermentation temperature that is lower than the typical fermentation temperature (*i.e.,* at approximately 35 ±1° C) for a short period of time within the fermentation process.

"*Clostridial* neurotoxin" means a neurotoxin produced from, or native to, a *Clostridial* bacterium, such as *Clostridium botulinum, Clostridium utyricum* or *Clostridium beratti,* as well as a *Clostridial* neurotoxin made recombinantly by a non-*Clostridial* species.

The term "carrier" used in connection with a pharmaceutical excipient refers to any and all solvents, dispersion media, preservatives, coatings, isotonic and absorption delaying agents, and the like, that are compatible with pharmaceutical administration.

The term "oxidized forms of BoNT/A" used herein refers to BoNT/A protein that is oxidized at at least one methionine residue. In one embodiment, the oxidized forms of BoNT/A in the compositions described herein comprise an oxidized methionine at one or more of positions 411 (M411) at SEQ ID NO: 2, and 550 (M550), 1004 (M1004), and 1144 (M1144) of SEQ ID NO: 3. The compositions described herein can comprise a plurality of oxidized forms of BoNT/A, each of which has a different oxidation profile (*i.e.,* oxidation at a different set of methionine residue(s)).

The term "oxidation level" used herein refers to the percentage of BoNT/A species within a composition having oxidation at a specific methionine residue of BoNT/A. The oxidation at the specific methionine residue of BoNT/A can be detected by methods known in the art, *e.g.,* by LC-MS/MS, and the percentage of oxidation at said methionine residue can be quantified using the following formula:

$$\% \text{ oxidation}_{(site\ n)} = \frac{(\text{peak area of the oxidized peptide})}{(\text{peak area of oxidized peptide} + \text{peak area unoxidized peptide})} \times 100$$

The term "patient", "subject", "individual" and the like refers to humans.

"Pharmaceutical composition" means a formulation in which an active ingredient can be a BoNT/A. The word "formulation" means that there is at least one additional ingredient (such as, for example and not limited to, an albumin (such as a human serum albumin (HSA) or a recombinant human albumin) and/or sodium chloride) in the pharmaceutical composition in addition to a BoNT/A active ingredient. The human serum albumin excipient can be derived from human plasma or recombinantly made. A pharmaceutical composition is therefore a formulation which is suitable for diagnostic, therapeutic and/or cosmetic administration (*e.g.*, by intramuscular or subcutaneous injection or by insertion of a depot or implant) to a subject, such as a human patient. A pharmaceutical composition can be in a lyophilized or vacuum dried condition, a solution reconstituted from the lyophilized or vacuum dried pharmaceutical composition with, for example, saline or water, or as a solution that does not require reconstitution. In one embodiment, the active ingredient is the *botulinum* toxin serotype A made natively by *Clostridial* bacteria. In one embodiment, the active ingredient is onabotulinumtoxinA. As stated, a pharmaceutical composition can be liquid or solid, for example vacuum-dried or lyophilized. Exemplary methods for formulating a BoNT/A active ingredient pharmaceutical composition are disclosed in U.S. Patent Application Publication No. 2003/0118598, filed Nov. 5, 2002, herein incorporated by reference in its entirety. In a preferred embodiment, a pharmaceutical composition described herein is in a dried form (*e.g.,* a vacuum-dried or lyophilized form). The pharmaceutical compositions can be vacuum-dried and suitable for administration by injection either subcutaneously or intramuscularly upon reconstitution with normal saline, comprising 900 kDa BoNT/A, human serum albumin (HSA), and sodium chloride. Preferably such pharmaceutical compositions comprise 0.5 mg of HSA and 0.9 mg of sodium chloride per 100 Units of BoNT/A. Most preferably such pharmaceutical compositions comprise 50, 100 or 200 Units of BoNT/A.

"Unit" or "U" refers to the $LD_{50}$ dose or the dose determined by a cell-based potency assay (CBPA). The $LD_{50}$ dose is defined as the amount of BoNT/A that killed 50% of the mice injected with the BoNT/A. The CBPA dose is determined as described in US Patent Nos. 8,618,261; 8,198,034; 9,249,216; 10,703,806; 11,261,240 and 11,332,518; the assay details of which are incorporated by reference herein.

Unless the context requires otherwise, the terms "comprise," "comprises," and "comprising" are used on the basis and clear understanding that they are to be interpreted inclusively, rather than exclusively, such that they indicate the inclusion of the recited feature but without excluding one or more other such features. However, it is understood that wherever aspects and embodiments are described herein with the language "comprise" (or "comprises" or "comprising"), otherwise analogous aspects described in terms of "consist of' (or "consists of" or "consisting of") and/or "consist essentially of" (or "consists essentially of" or "consisting essentially of") are also provided.

### 5.2. *Clostridium botulinum* neurotoxin serotype A (BoNT/A)

In one aspect, the composition comprises 150-kDa *Clostridium botulinum* neurotoxin serotype A (BoNT/A) in both oxidized and unoxidized forms.

A 150 kDa BoNT/A molecule is a zinc endopeptidase which can specifically hydrolyze a peptide linkage of the intracellular, vesicle-associated protein (VAMP, also called synaptobrevin) 25 kiloDalton (kDa) synaptosomal associated protein (SNAP-25).

A 150 kDa BoNT/A molecule is translated as a single chain polypeptide of approximately 150 kDa that is subsequently cleaved by proteolytic scission within a disulfide loop by a naturally-occurring protease. This posttranslational processing yields a di-chain molecule comprising an approximately 50 kDa light chain (LC) and an approximately 100 kDa heavy chain (HC) held together by a single disulfide bond and noncovalent interactions. *Clostridial* bacterium can produce *botulinum* toxin type A complexes in various forms, which include, and are not limited to, 900 kDa, 760 kDa, 500 kDa, and 300 kDa complexes (approximate molecular weights). The complexes (*i.e.,* molecular weight greater than about 150 kDa) comprise a 150 kDa *botulinum* toxin molecule (the neurotoxic component) and one or more hemagglutinin (HA) proteins and/or non-toxin non-hemagglutinin (NTNH) protein.

In one embodiment, the 150 kDa BoNT/A molecule that can be used in the context of the present disclosure has a sequence shown in Table 1. In one embodiment, the 150 kDa BoNT/A molecule comprises a light chain (LC: residues 2-438, about 50 kDa) and a heavy chain (HC: residues 449-1296, about 100 kDa) . Residues 439-448 are the nicking site and are italicized. The oxidation sites M411, M550, M1004, and M1144 are underlined. The amino acid residue positions recited in various claims, embodiments and aspects of this disclosure refer to the amino acid residue positions depicted in Table 1. According to Table 1, the first amino acid residue of SEQ ID NO: 2 starts from position 2 of SEQ ID NO: 2 rather than position 1 of SEQ ID NO: 2, and the first amino acid residue of SEQ ID NO: 3 starts from position 449 of SEQ ID NO: 3 rather than position 1 of SEQ ID NO: 3.

Table 1. Preferred sequences of BoNT/A molecules.

SEQ ID NO. 1: amino acid sequence of the 150 kDa BoNT/A molecule

```
MPFVNKQFNY  KDPVNGVDIA  YIKIPNAGQM  QPVKAFKIHN  KIWVIPERDT  50
FTNPEEGDLN  PPPEAKQVPV  SYYDSTYLST  DNEKDNYLKG  VTKLFERIYS  100
TDLGRMLLTS  IVRGIPFWGG  STIDTELKVI  DTNCINVIQP  DGSYRSEELN  150
LVIIGPSADI  IQFECKSFGH  EVLNLTRNGY  GSTQYIRFSP  DFTFGFEESL  200
EVDTNPLLGA  GKFATDPAVT  LAHELIHAGH  RLYGIAINPN  RVFKVNTNAY  250
YEMSGLEVSF  EELRTFGGHD  AKFIDSLQEN  EFRLYYYNKF  KDIASTLNKA  300
KSIVGTTASL  QYMKNVFKEK  YLLSEDTSGK  FSVDKLKFDK  LYKMLTEIYT  350
EDNFVKFFKV  LNRKTYLNFD  KAVFKINIVP  KVNYTIYDGF  NLRNTNLAAN  400
FNGQNTEINN  MNFTKLKNFT  GLFEFYKLLC  VRGIITSKTK  SLDKGYNK
                                                     AL  450
NDLCIKVNNW  DLFFSPSEDN  FTNDLNKGEE  ITSDTNIEAA  EENISLDLIQ  500
QYYLTFNFDN  EPENISIENL  SSDIIGQLEL  MPNIERFPNG  KKYELDKYTM  550
FHYLRAQEFE  HGKSRIALTN  SVNEALLNPS  RVYTFFSSDY  VKKVNKATEA  600
AMFLGWVEQL  VYDFTDETSE  VSTTDKIADI  TIIIPYIGPA  LNIGNMLYKD  650
DFVGALIFSG  AVILLEFIPE  IAIPVLGTFA  LVSYIANKVL  TVQTIDNALS  700
KRNEKWDEVY  KYIVTNWLAK  VNTQIDLIRK  KMKEALENQA  EATKAIINYQ  750
YNQYTEEEKN  NINFNIDDLS  SKLNESINKA  MININKFLNQ  CSVSYLMNSM  800
IPYGVKRLED  FDASLKDALL  KYIYDNRGTL  IGQVDRLKDK  VNNTLSTDIP  850
FQLSKYVDNQ  RLLSTFTEYI  KNIINTSILN  LRYESNHLID  LSRYASKINI  900
GSKVNFDPID  KNQIQLFNLE  SSKIEVILKN  AIVYNSMYEN  FSTSFWIRIP  950
KYFNSISLNN  EYTIINCMEN  NSGWKVSLNY  GEIIWTLQDT  QEIKQRVVFK  1000
YSQMINISDY  INRWIFVTIT  NNRLNNSKIY  INGRLIDQKP  ISNLGNIHAS  1050
NNIMFKLDGC  RDTHRYIWIK  YFNLFDKELN  EKEIKDLYDN  QSNSGILKDF  1100
WGDYLQYDKP  YYMLNLYDPN  KYVDVNNVGI  RGYMYLKGPR  GSVMTTNIYL  1150
NSSLYRGTKF  IIKKYASGNK  DNIVRNNDRV  YINVVVKNKE  YRLATNASQA  1200
GVEKILSALE  IPDVGNLSQV  VVMKSKNDQG  ITNKCKMNLQ  DNNGNDIGFI  1250
GFHQFNNIAK  LVASNWYNRQ  IERSSRTLGC  SWEFIPVDDG  WGERPL      1296
```

6

(continued)

## SEQ ID NO. 2: amino acid sequence of the light chain (LC) of BoNT/A molecule

```
PFVNKQFNY KDPVNGVDIA YIKIPNAGQM QPVKAFKIHN KIWVIPERDT 50
FTNPEEGDLN PPPEAKQVPV SYYDSTYLST DNEKDNYLKG VTKLFERIYS 100
TDLGRMLLTS IVRGIPFWGG STIDTELKVI DTNCINVIQP DGSYRSEELN 150
LVIIGPSADI IQFECKSFGH EVLNLTRNGY GSTQYIRFSP DFTFGFEESL 200
EVDTNPLLGA GKFATDPAVT LAHELIHAGH RLYGIAINPN RVFKVNTNAY 250
YEMSGLEVSF EELRTFGGHD AKFIDSLQEN EFRLYYYNKF KDIASTLNKA 300
KSIVGTTASL QYMKNVFKEK YLLSEDTSGK FSVDKLKFDK LYKMLTEIYT 350
EDNFVKFFKV LNRKTYLNFD KAVFKINIVP KVNYTIYDGF NLRNTNLAAN 400
FNGQNTEINN MNFTKLKNFT GLFEFYKLLC VRGIITSK
```

## SEQ ID NO. 3: amino acid sequence of the heavy chain (HC) of BoNT/A molecule

```
                                                   AL 450
NDLCIKVNNW DLFFSPSEDN FTNDLNKGEE ITSDTNIEAA EENISLDLIQ 500
QYYLTFNFDN EPENISIENL SSDIIGQLEL MPNIERFPNG KKYELDKYTM 550
FHYLRAQEFE HGKSRIALTN SVNEALLNPS RVYTFFSSDY VKKVNKATEA 600
AMFLGWVEQL VYDFTDETSE VSTTDKIADI TIIIPYIGPA LNIGNMLYKD 650
DFVGALIFSG AVILLEFIPE IAIPVLGTFA LVSYIANKVL TVQTIDNALS 700
KRNEKWDEVY KYIVTNWLAK VNTQIDLIRK KMKEALENQA EATKAIINYQ 750
YNQYTEEEKN NINFNIDDLS SKLNESINKA MININKFLNQ CSVSYLMNSM 800
IPYGVKRLED FDASLKDALL KYIYDNRGTL IGQVDRLKDK VNNTLSTDIP 850
FQLSKYVDNQ RLLSTFTEYI KNIINTSILN LRYESNHLID LSRYASKINI 900
GSKVNFDPID KNQIQLFNLE SSKIEVILKN AIVYNSMYEN FSTSFWIRIP 950
KYFNSISLNN EYTIINCMEN NSGWKVSLNY GEIIWTLQDT QEIKQRVVFK 1000
YSQMINISDY INRWIFVTIT NNRLNNSKIY INGRLIDQKP ISNLGNIHAS 1050
NNIMFKLDGC RDTHRYIWIK YFNLFDKELN EKEIKDLYDN QSNSGILKDF 1100
WGDYLQYDKP YYMLNLYDPN KYVDVNNVGI RGYMYLKGPR GSVMTTNIYL 1150
NSSLYRGTKF IIKKYASGNK DNIVRNNDRV YINVVVKNKE YRLATNASQA 1200
GVEKILSALE IPDVGNLSQV VVMKSKNDQG ITNKCKMNLQ DNNGNDIGFI 1250
GFHQFNNIAK LVASNWYNRQ IERSSRTLGC SWEFIPVDDG WGERPL     1296
```

In one embodiment, the BoNT/A described herein is present as a 900 kDa BoNT/A complex. In one embodiment, the BoNT/A described herein is present as a 900 kDa BoNT/A complex formed by the 150 kDa BoNT/A molecule and hemagglutinin HA70, hemagglutinin HA34, hemagglutinin HA17, and nontoxic-nonhemagglutinin (NTNH) proteins. In a specific embodiment, the BoNT/A described herein (*e.g.,* the 900 kDa BoNT/A complex) is produced in a *Clostridium botulinum* type A strain. In a specific embodiment, the BoNT/A described herein (*e.g.,* the 900 kDa BoNT/A complex) is produced in a *Clostridium botulinum* type A Hall strain of *Clostridium botulinum.* In a preferred embodiment, the BoNT/A described herein is onabotulinumtoxinA.

In one embodiment, the 150 kDa BoNT/A molecule described herein comprises a light chain having an amino acid sequence set forth in SEQ ID NO. 2 and a heavy chain having an amino acid sequence set forth in SEQ ID NO. 3, with disulfide bridges located between positions 429 and 453 and between positions 1234 and 1279.

### 5.3. Oxidized BoNT/A Compositions

In one embodiment, the present disclosure relates to a composition comprising a plurality of 900 kDa *Clostridium botulinum* serotype A (BoNT/A) neurotoxin complex species, wherein the oxidation level of 150 kDa neurotoxin species present in the composition is less than 6% at each of the following positions: 411 (M411) as shown in SEQ ID NO:2, 550 (M550) as show in SEQ ID NO:3, 1004 (M1004) as shown in SEQ ID NO:3, and 1144 (M1144) as shown in SEQ ID NO:3. For example, and without limitation, the oxidation level at one or more of said positions is less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%, and ranges and amounts between any of these aforementioned oxidation percentages.

In one embodiment, the oxidation level at each of positions 411 (M411) of SEQ ID NO: 2, and 550 (M550), 1004 (M1004), and 1144 (M1144) of SEQ ID NO: 3 is less than 6.0%. For example, and without limitation, the oxidation level at each said position is less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%.

In one embodiment, the oxidized forms of BoNT/A in the compositions described herein comprise an oxidized methionine at position 411 (M411) of SEQ ID NO. 2.

In one embodiment, the oxidation level at position M411 of SEQ ID NO. 2 is less than *5% (e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%).

In one embodiment, the oxidation level at position M411 of SEQ ID NO. 2 is about 1% to about 5%, about 1% to about 4%, about 1% to about 3%, about 1% to about 2%, about 2% to about 5%, about 2% to about 4%, about 2% to about 3%, or about 3% to about 5%, or about 4% to about 5%.

In one embodiment, the oxidation level at position M411 of SEQ ID NO. 2 is about 2%.

In one embodiment, the oxidized forms of BoNT/A in the compositions described herein comprise an oxidized methionine at position 550 (M550) of SEQ ID NO. 3.

In one embodiment, the oxidation level at position M550 of SEQ ID NO. 3 is less than 1% (*e.g.,* less than 0.8%, less than 0.6%, less than 0.5%, less than 0.4%, less than 0.3%, less than 0.2%, or less than 0.1%).

In one embodiment, the oxidation level at position M550 of SEQ ID NO. 3 is about 0.3% to about 0.5% (*e.g.,* about 0.3%, about 0.35%, about 0.4%, about 0.45%, or about 0.5%).

In one embodiment, the oxidized forms of BoNT/A in the compositions described herein comprise an oxidized methionine at position 1004 (M1004) of SEQ ID NO. 3.

In one embodiment, the oxidation level at position M1004 of SEQ ID NO. 3 is less than *3% (e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M1004 of SEQ ID NO. 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%).

In one embodiment, the oxidized forms of BoNT/A in the compositions described herein comprise an oxidized methionine at position 1144 (M1144) of SEQ ID NO. 3.

In one embodiment, the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M1144 of SEQ ID NO. 3 is about 1% to about 5%, about 1% to about 4%, about 1% to about 3%, about 1% to about 2%, about 2% to about 5%, about 2% to about 4%, about 2% to about 3%, or about 3% to about 5%, or about 4% to about 5%.

In one embodiment, the oxidation level at position M1144 of SEQ ID NO. 3 is about 2%.

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), and the oxidation level at position M550 of SEQ ID NO: 3 is less than 1% (*e.g.,* less than 0.8%, less than 0.6%, less than 0.5%, less than 0.4%, less than 0.3%, less than 0.2%, or less than 0.1%).

In one embodiment, the oxidation level at position M411 of SEQ ID NO. 2 is about 2% and the oxidation level at position M550 of SEQ ID NO: 3 is about 0.3% to about 0.5% (*e.g.,* about 0.3%, about 0.35%, about 0.4%, about 0.45%, or about 0.5%).

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), and the oxidation level at position M1004 of SEQ ID NO: 3 is less than 3% (*e.g., e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M411 of SEQ ID NO. 2 is about 2% and the oxidation level at position M1004 of SEQ ID NO: 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%).

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), and the oxidation level at position M1144 of SEQ ID NO: 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M411 of SEQ ID NO. 2 is about 2% and the oxidation level at position M1144 of SEQ ID NO: 3 is about 2%.

In one embodiment, the oxidation level at position M550 SEQ ID NO: 3 is less than 1% (*e.g.,* less than 0.8%, less than 0.6%, less than 0.5%, less than 0.4%, less than 0.3%, less than 0.2%, or less than 0.1%), and the oxidation level at position M1004 of SEQ ID NO: 3 is less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M550 of SEQ ID NO: 3 is about 0.3% to about 0.5% (*e.g.,* about 0.3%, about 0.35%, about 0.4%, about 0.45%, or about 0.5%) and the oxidation level at position M1004 of SEQ ID NO: 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%).

In one embodiment, the oxidation level at position M550 of SEQ ID NO. 3 is less than 1% (*e.g.,* less than 0.8%, less than 0.6%, less than 0.5%, less than 0.4%, less than 0.3%, less than 0.2%, or less than 0.1%), and the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M550 of SEQ ID NO. 3 about 0.3% to about 0.5% (*e.g.,* about 0.3%, about 0.35%, about 0.4%, about 0.45%, or about 0.5%), and the oxidation level at position M1144 of SEQ ID NO. 3 is about 2%.

In one embodiment, the oxidation level at position M1004 of SEQ ID NO. 3 is less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%), and the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M1004 of SEQ ID NO. 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%), and the oxidation level at position M1144 of SEQ ID NO. 3 is about 2%.

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), the oxidation level at position M550 of SEQ ID NO. 3 is less than 1% (*e.g.,* less than 0.8%, less than 0.6%, less than 0.5%, less than 0.4%, less than 0.3%, less than 0.2%, or less than 0.1%), and the oxidation level at position M1004 of SEQ ID NO. 3 is less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M411 of SEQ ID NO. 2 is about 2%, the oxidation level at position M550 of SEQ ID NO. 3 is about 0.3% to about 0.5% (*e.g.,* about 0.3%, about 0.35%, about 0.4%, about 0.45%, or about 0.5%), and the oxidation level at position M1004 of SEQ ID NO. 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%).

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), the oxidation level at position M550 of SEQ ID NO. 3 is less than 1% (*e.g.,* less than 0.8%, less than 0.6%, less than 0.5%, less than 0.4%, less than 0.3%, less than 0.2%, or less than 0.1%), and the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M411 of SEQ ID NO. 2 is about 2%, the oxidation level at position M550 of SEQ ID NO. 3 is about 0.3% to about 0.5% (*e.g.,* about 0.3%, about 0.35%, about 0.4%, about 0.45%, or about 0.5%), and the oxidation level at position M1144 of SEQ ID NO. 3 is about 2%.

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), the oxidation level at position M1004 of SEQ ID NO. 3 is less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%), and the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M411 of SEQ ID NO. 2 is about 2%, the oxidation level at position M1004 of SEQ ID NO. 3 is about 1% to about 2% (*e.g.*, about 1%, about 1.1%, about 1.2%, about 1.3%, about 1.4%, about 1.5%, about 1.6%, about 1.7%, about 1.8%, about 1.9% or about 2%), and the oxidation level at position M1144 of SEQ ID NO. 3 is about 2%.

In one embodiment, the oxidation level at position M550 of SEQ ID NO. 3 is less than 1% (*e.g.,* less than 0.8%, less than 0.6%, less than 0.5%, less than 0.4%, less than 0.3%, less than 0.2%, or less than 0.1%), the oxidation level at position M1004 of SEQ ID NO. 3 is less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%), and the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M550 of SEQ ID NO. 3 is about 0.3% to about 0.5% (*e.g.,* about 0.3%, about 0.35%, about 0.4%, about 0.45%, or about 0.5%), the oxidation level at position M1004 of SEQ ID NO. 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%), and the oxidation level at position M1144 of SEQ ID NO. 3 is about 2%.

In one embodiment, the oxidation level at position M411 of SEQ ID NO. 2 is less than 5% (*e.g.,* less than 4.5%, less than 4%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, less than 0.1%), the oxidation level at position M550 of SEQ ID NO. 3 is less than 1% (*e.g.,* less than 0.8%, less than 0.6%, less than 0.5%, less than 0.4%, less than 0.3%, less than 0.2%, or less than 0.1%), the oxidation level at position M1004 of SEQ ID NO. 3 is less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%), and the oxidation level at position M1144 of SEQ ID NO. 3 is less than 6% (*e.g.,* less than 5.5%, less than 5.0%, less than 4.5%, less than 4.0%, less than 3.5%, less than 3%, less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%).

In one embodiment, the oxidation level at position M411 of SEQ ID NO. 2 about 2%, the oxidation level at position M550 of SEQ ID NO. 3 is about 0.3% to about 0.5% (*e.g.,* about 0.3%, about 0.35%, about 0.4%, about 0.45%, or about 0.5%), the oxidation level at position M1004 of SEQ ID NO. 3 is about 1% to about 2% (*e.g.,* about 1%, about 1.1%, about 1.2%, about 1.3%, about 1.4%, about 1.5%, about 1.6%, about 1.7%, about 1.8%, about 1.9% or about 2%), and the oxidation level at position M1144 of SEQ ID NO. 3 is about 2%.

The oxidation level of BoNT/A compositions described herein can be determined using methods known in the art, *e.g.,* by mass spectrometry. In one embodiment, the oxidation level is determined by liquid chromatography with tandem mass spectrometry (LC-MS/MS), for example, without limitation, by using the LC-MS/MS method illustrated in the Example 2.

In various aspects and embodiments, the composition described herein further comprises human serum albumin (HSA). In certain embodiments, the composition comprises about 0.5 mg of HSA per 100 units of the 900 kDa BoNT/A complex. In certain embodiments, the HSA is recombinant HSA. In specific embodiments, the recombinant HSA is animal product free. In specific embodiments, the recombinant HSA is not produced from an animal. In specific embodiments, the recombinant HSA is produced from a microorganism, such as bacteria. In specific embodiments, the recombinant HSA is produced from a plant-based expression system. In certain embodiments, the HSA is human plasma-derived.

In various aspects and embodiments, the composition described herein further comprises sodium chloride. In certain embodiments, the composition comprises about 0.9 mg of sodium chloride per 100 units of the 900 kDa BoNT/A complex.

In various aspects and embodiments, the composition described herein further comprises a pharmaceutically acceptable carrier. In various aspects and embodiments, the pharmaceutically acceptable carrier comprises human serum albumin and sodium chloride.

In various aspects and embodiments, the composition described herein is a liquid composition. In various aspects and embodiments, the composition described herein is a solid composition. In various aspects and embodiments, the composition described herein is a vacuum-dried composition. In certain embodiments, the composition described herein is a freeze-dried composition. In certain embodiments, the composition described herein is a powdered pharmaceutical composition. In specific embodiments, the composition described herein is a drug product. In a specific embodiment, the composition described herein is a drug product and further comprises HSA (*e.g.*, about 0.5 mg of HSA per 100 units of the 900 kDa BoNT/A complex) and sodium chloride (*e.g.*, about 0.9 mg of sodium chloride per 100 units of the 900 kDa BoNT/A complex). In specific embodiments, the composition described herein is a drug substance. In a specific embodiment, the composition described herein is a drug substance in a solution suitable for storage.

In various aspects and embodiments, the composition described herein is animal product free. In various aspects and embodiments, the composition described herein does not contain a protease inhibitor. In certain embodiments, the composition described herein does not contain benzamidine hydrocholoride. In various aspects and embodiments, the 900 kDa BoNT/A complex is produced by *Clostridium botulinum* bacteria cultured and expanded from an animal product free working cell bank. The animal product free working cell bank can be produced, for example and without limitation, as described in Example 6.

In various aspects and embodiments, the composition described herein comprises about 50 units of the 900 kDa BoNT/A complex. In various aspects and embodiments, the composition described herein comprises about 100 units of the 900 kDa BoNT/A complex. In various aspects and embodiments, the composition described herein comprises about 200 units of the 900 kDa BoNT/A complex.

In various aspects and embodiments, the 900 kDa BoNT/A complex is produced in a fermentation condition comprising a cold shock fermentation temperature.

In various aspects and embodiments, the 900 kDa BoNT/A complex is produced in a continuous cold temperature fermentation condition.

In various aspects and embodiments, the 900 kDa BoNT/A complex is produced using a fermentation condition (*e.g.*, a fermentation temperature setting) as described in Section 5.4.

In various aspects and embodiments, the 900 kDa BoNT/A complex is produced using a fermentation condition (*e.g.,* a fermentation temperature setting) as described in Example 1.

In various aspects and embodiments, the 900 kDa BoNT/A complex is produced as described in Section 5.4.

In various aspects and embodiments, the 900 kDa BoNT/A complex is produced as described in Example 1.

In various aspects and embodiments, the 900 kDa BoNT/A complex is produced in a *Clostridium botulinum* type A strain of *Clostridium botulinum* (*e.g.,* the Type A Hall strain of *Clostridium botulinum*). In specific embodiments, the 900 kDa BoNT/A complex is onabotulinumtoxin A.

In various aspects and embodiments, the 900 kDa BoNT/A complex is purified by a chromatographic process (*e.g.*, a chromatographic process described in Section 5.4).

In one embodiment, the present disclosure also relates to a method of treating a patient (preferably, a human patient) in need thereof, comprising administering a composition described herein.

## 5.4. Production of BoNT/A and Production of BoNT/A Compositions

*Botulinum* toxin type A has been approved by the U.S. Food and Drug Administration (FDA) for the treatment of essential blepharospasm, strabismus and hemifacial spasm in patients over the age of twelve, cervical dystonia, glabellar line (facial) wrinkles and for treating hyperhydrosis. A commercially available *botulinum* toxin type A containing pharmaceutical composition is sold under the trademark BOTOX® (onabotulinumtoxinA), available commercially from Allergan, an AbbVie company, North Chicago, Illinois, USA. BOTOX® contains a purified 900 kDa *botulinum* toxin type A complex, human serum albumin, and sodium chloride packaged in sterile, vacuum-dried form. The *botulinum* toxin type A complex in BOTOX® is made from a culture of the Hall strain of *Clostridium botulinum* grown in a medium containing N-Z amine casein and yeast extract (*i.e.,* non-APF process) and purified from the culture solution by a series of precipitation (including acid precipitation) steps to a crystalline complex consisting of the active high molecular weight toxin protein and an associated hemagglutinin protein. The crystalline complex is re-dissolved in a solution containing saline and albumin and sterile filtered using a gamma-irradiated filter (0.2 microns) prior to vacuum-drying. BOTOX® can be reconstituted with sterile, non-preserved saline prior to intramuscular injection. Each 100 unit vial of BOTOX® consists of about 5 ng of purified *botulinum* toxin type A complex, 0.5 mg human serum albumin, and 0.9 mg sodium chloride, vacuum-dried form and intended for reconstitution with sterile normal saline without a preservative (0.9% sodium chloride injection).

A number of steps are required to make the BoNT/A compositions described herein suitable for administration to a human or animal for a therapeutic, diagnostic, research or cosmetic purpose. In one embodiment, these steps can include obtaining a purified *Clostridium botulinum* neurotoxin serotype A (BoNT/A) with both oxidized and unoxidized forms and then compounding the purified BoNT/A. A first step can be to culture a *Clostridial* bacteria (*e.g.,* the Hall strain of *Clostridium botulinum*), typically on agar plates, in an environment conducive to bacterial growth, such as in a warm anaerobic atmosphere. The culture step using agar plates allows *Clostridial* colonies with desirable morphology and other characteristics to be obtained. The culture step can also be performed with bacteria from an animal product free working cell back (*see, e.g.,* Example 6). In a second step, selected cultured *Clostridial* colonies can be fermented in a suitable medium. After a certain period of fermentation the *Clostridial* bacteria typically lyse and release *Clostridial* toxin (*e.g.,* BoNT/A) into the medium. Thirdly, the toxin can be purified from the culture medium to obtain a bulk or raw BoNT/A toxin drug substance. Preferably, the BoNT/A toxin drug substance will not have been subjected to precipitation (*e.g.,* precipitation with cold ethanol, hydrochloric acid, and/or ammonium sulfate), *e.g.,* during purification. Also preferred is BoNT/A toxin drug substance that has been purified by column chromatography, particularly BoNT/A toxin drug substance produced by purification using a hydrophobic interaction chromatography (HIC) column. When multiple chromatography columns are used to purify the BoNT/A, it is preferable that the toxin is purified using a process wherein a HIC column is used prior to all other chromatography columns. It is preferable that BoNT/A drug substance is produced by a process that qualifies for use under the Good Manufacturing Practice regulations promulgated by the U.S. Food and Drug Administration.

In some embodiments, the BoNT/A compositions described herein are obtained using a substantially, essentially or entirely animal protein free (APF) process. The process can comprise the following sequential steps: culturing *Clostridium botulinum* bacteria (*e.g.,* the Hall strain of *Clostridium botulinum*) in a substantially APF culture medium; fermenting *Clostridium botulinum* bacteria from the culture medium in a substantially APF fermentation medium, harvesting the fermentation medium by removing cellular debris present in the fermentation medium using filtration or centrifugation; concentrating the harvested fermentation medium by filtration, such as by ultrafiltration (UF); diluting the concentrated fermentation medium by adding a buffer. Following dilution with the buffer, a substantially APF chromatographic process can be undertaken to obtain biologically active highly purified BoNT/A complex. In some embodiments, the APF process described herein does not involve using a protease inhibitor. In some embodiments, the APF process described herein does not involve using benzamidine hydrochloride. In one embodiment, the BoNT/A compositions described herein are obtained using the APF process disclosed in Example 1.

The substantially APF culture and/or fermentation medium can contain a protein product obtained from yeast (*e.g.,* yeast extract or yeast extract concentrate), or from a vegetable (*e.g.,* wheat, soy, malt, Lupinus, corn, cottonseed, *L. campestri* seed, etc.). The APF culture and/or fermentation medium can further comprise a carbon source (*e.g.,* glucose) and/or a source of salt (*e.g.,* sodium chloride). In one embodiment, the medium used for fermentation of *Clostridium botulinum* is free of animal by-products and comprises approximately 10-100 g/L (*e.g.,* 20-60 g/L) hydrolyzed soy (Hy-Soy), approximately 7.5 g/L glucose, and 5.0 g/L NaCl, as disclosed in U.S. Pat. No. 7,354,740, which is incorporated by reference herein in its entirety. In one embodiment, the fermentation medium can comprise 3% w/v or 5% w/v HySoy; 1% w/v HyYeast; and 1% w/v glucose, as disclosed in U.S. Pat. No. 8,129,139, which is incorporated by reference herein in its entirety. In one embodiment, the APF fermentation medium can comprise 3.25 % w/v soy peptone type II, 1.2 % w/v yeast extract, 1.5 % w/v glucose, pH adjusted to 7.3 using sodium hydroxide, as illustrated in Example 1 below.

In some embodiments, the BoNT/A compositions described herein are obtained under a cold shock condition, *i.e.,* at a temperature that is lower than the typical fermentation temperature (*i.e.,* at approximately 35 $\pm$1 °C) for a short period of time within the fermentation process. In some embodiments, the BoNT/A compositions are obtained under cold shock conditions at about 33 °C, 30 °C, 27 °C, 25 °C, 22 °C, 20 °C, 19 °C, 18 °C, 17 °C, 16 °C, or 15 °C. The cold shock conditions can last for any period of time within the entire fermentation process, for example, for about 2 hours, 5 hours, 8 hours, or 10 hours within a 72-hour fermentation process. The cold shock conditions can occur once or multiple times during the fermentation process. In some embodiments, the BoNT/A compositions described herein are obtained using about 15 °C-20 °C (*e.g.,* about 15 °C, 16 °C, 17 °C, 18 °C, 19 °C, or 20 °C) cold shock for about 5 hours (*e.g.,* from the 13th to 17th hour in a 72-hour fermentation process) during fermentation.

In some embodiments, the BoNT/A compositions described herein are obtained under a continuous cold condition, *i.e.,* at a temperature that is continuously lower (*e.g.,* about 25 °C) than the typical fermentation temperature (*i.e.,* at approximately 35 $\pm$1 °C) throughout the entire fermentation process. In some embodiments, the BoNT/A compositions are obtained under a continuous cold condition at about 33 °C, 30 °C, 27 °C, 25 °C, 22 °C, 20 °C, 19 °C, 18 °C, 17 °C, 16 °C, or 15° C. In some embodiments, the BoNT/A compositions described herein are obtained by fermenting a culture of *Clostridium botulinum* at about 25° C for about 160 hours.

In one aspect, provided herein is a method of producing a composition comprising a 900 kDa BoNT/A complex (such as onabotulinumtoxinA), said method comprising incubating a culture of *Clostridium botulinum* bacteria (*e.g., Clostridium botulinum* type A Hall strain bacteria) in a production fermentor at a temperature that is below 35 °C for a period of time.

In certain embodiments, the entire fermentation period is about 60 hours to about 200 hours. In certain embodiments, the entire fermentation period is about 60 hours to about 160 hours. In certain embodiments, the entire fermentation period is about 60 hours to about 120 hours. In certain embodiments, the entire fermentation period is about 60 hours to about 80 hours. In certain embodiments, the entire fermentation period is about 68 hours to about 76 hours. In certain embodiments, the entire fermentation period is about 65 hours to about 72 hours. In certain embodiments, the entire fermentation period is about 72 hours. In certain embodiments, the entire fermentation period is about 120 hours to about 200 hours. In certain embodiments, the entire fermentation period is about 120 hours to about 180 hours. In certain embodiments, the entire fermentation period is about 140 hours to about 180 hours. In certain embodiments, the entire fermentation period is about 160 hours.

In certain embodiments, the period of time (for low temperature incubation) is about 2 hours. In certain embodiments, the period of time is about 5 hours. In certain embodiments, the period of time is about 8 hours. In certain embodiments, the period of time is about 10 hours. In certain embodiments, the period of time is about 2-5 hours. In certain embodiments, the period of time is about 5-8 hours. In certain embodiments, the period of time is about 8-10 hours. In certain embodiments, the period of time is the entire fermentation period.

In certain embodiments, the temperature is about 33 °C, 32 °C, 31 °C, 30 °C, 29 °C, 28 °C, 27 °C, 26 °C, 25 °C, 24 °C, 23 °C, 22 °C, 21 °C, 20 °C, 19 °C, 18 °C, 17 °C, 16 °C, or 15 °C. In certain embodiments, the temperature is about 15 °C-20 °C (*e.g.,* about 15 °C, 16 °C, 17 °C, 18 °C, 19 °C, or 20 °C). In certain embodiments, the temperature is about 15 °C. In certain embodiments, the temperature is about 20 °C. In certain embodiments, the temperature is about 25 °C.

In certain embodiments, the method comprises, in the following order: (a) incubating the culture of *Clostridium botulinum* bacteria in the production fermentor at 35 °C for about 12 hours; (b) setting temperature to about 15 °C -20 °C (*e.g.,* about 15 °C, 16 °C, 17 °C, 18 °C, 19 °C, or 20 °C) at the end of step (a); (c) culturing at the set temperature until about 5 hours after step (a); (d) setting temperature to 35 °C at the end of step (c); and (e) culturing at 35 °C until about 55 hours after step (c).

In certain embodiments, the method comprises incubating the culture of *Clostridium botulinum* bacteria in the production fermentor at 25 °C for about 160 hours.

In certain embodiments, the method is performed as described in Example 1.

In various aspects and embodiments, the fermentation is performed in animal protein free cell culture media.

An animal product free or substantially animal product free chromatographic system and process can be used to purify a clarified culture of *Clostridium botulinum* obtained from the APF fermentation processes described herein. The chromatographic system and process can include one column, two columns or three columns. For example, the chromatographic system and process can comprise one, two, or three steps: a hydrophobic interaction chromatography (HIC), and/or an anion exchange chromatography (AEX), and/or a cation exchange chromatograph. In some embodiments, the chromatographic process comprises a first step of subjecting the BoNT/A culture obtained from the APF process to a HIC, followed by subjecting a BoNT/A-containing eluent from HIC to an AEX, and then followed by subjecting the BoNT/A-containing captured solution from AEX to a CEX. In some embodiments, the chromatography-based purification process further comprises processing the eluent from the columns by diafiltration (DF) and filtering the processed eluent. In some embodiments, the chromatography-based purification process is as disclosed in U.S. Pat. No. 8,129,139, which is incorporated by reference herein in its entirety.

The BoNT/A complex obtained from the APF fermentation and purification processes described herein is biologically active and highly purified. In one embodiment, the BoNT/A compositions described herein comprise less than 3% (*e.g.,* less than 2.5%, less than 2.0%, less than 1.8%, less than 1.5%, less than 1.2%, less than 1.0%, less than 0.8%, less than 0.6%, less than 0.5%, less than 0.4%, less than 0.3%, less than 0.2%, or less than 0.1%) of host cell protein. The level of the host cell protein can be assessed using methods known in the art, *e.g.,* using Size Exclusion High Performance Liquid Chromatography (SEC-HPLC) or SEC-HPLC in conjugation with Multi-Angle Laser Light Scattering (MALLS) as disclosed in Lietzow et al. (2008) Protein J 27:420-425, which is incorporated herein by reference in its entirety. In one embodiment, the percentage of the host cell protein in the BoNT/A compositions described herein is determined using SEC-HPLC, *e.g.,* using the SEC-HPLC method as illustrated in Example 3.

In various aspects and embodiments, the 900 kDa BoNT/A complex is produced by a Type A strain of *Clostridium botulinum* (*e.g.,* the Type A Hall strain of *Clostridium botulinum).* In specific embodiments, the 900 kDa BoNT/A complex is onabotulinumtoxin A.

In various aspects and embodiments, the 900 kDa BoNT/A complex is produced as described in Example 5, Example 6 or Example 7.

In various aspects and embodiments, the 900 kDa BoNT/A complex is produced using one or more steps as described in Example 5, Example 6 or Example 7.

In various aspects and embodiments, the 900 kDa BoNT/A complex is produced by a process that comprises one or more steps of column chromatography performed during purification of the 900 kDa BoNT/A complex.

In preferred embodiments, the one or more steps of column chromatography performed during purification of the 900 kDa BoNT/A complex comprise hydrophobic interaction chromatography. In specific embodiments, the one or more steps of column chromatography comprise anion exchange chromatography. In specific embodiments, the one or more steps of column chromatography comprise cation exchange chromatography.

In specific embodiments, the one or more steps of column chromatography performed during purification of the 900 kDa BoNT/A complex comprise hydrophobic interaction chromatography and anion exchange chromatography. In a specific embodiment, the one or more steps of column chromatography comprise, in the following order, hydrophobic interaction chromatography and anion exchange chromatography. In a specific embodiment, the one or more steps of column chromatography comprise, in the following order, anion exchange chromatography and hydrophobic interaction chromatography.

In specific embodiments, the one or more steps of column chromatography performed during purification of the 900 kDa BoNT/A complex comprise hydrophobic interaction chromatography and cation exchange chromatography. In a specific embodiment, the one or more steps of column chromatography comprise, in the following order, hydrophobic interaction chromatography and cation exchange chromatography. In a specific embodiment, the one or more steps of column chromatography comprise, in the following order, cation exchange chromatography and hydrophobic interaction chromatography.

In specific embodiments, the one or more steps of column chromatography performed during purification of the 900 kDa BoNT/A complex comprise anion exchange chromatography and cation exchange chromatography. In a specific embodiment, the one or more steps of column chromatography comprise, in the following order, anion exchange chromatography and cation exchange chromatography. In a specific embodiment, the one or more steps of column chromatography comprise, in the following order, cation exchange chromatography and anion exchange chromatography.

In specific embodiments, the one or more steps of column chromatography performed during purification of the 900 kDa BoNT/A complex comprise hydrophobic interaction chromatography, anion exchange chromatography, and cation exchange chromatography. In a preferred embodiment, the one or more steps of column chromatography comprise, in the following order, hydrophobic interaction chromatography, anion exchange chromatography, and cation exchange chromatography. In a specific embodiment, the one or more steps of column chromatography comprise, in the following order, hydrophobic interaction chromatography, cation exchange chromatography, and anion exchange chromatography. In a specific embodiment, the one or more steps of column chromatography comprise, in the following order, anion exchange chromatography, cation exchange chromatography, and hydrophobic interaction chromatography. In a specific embodiment, the one or more steps of column chromatography comprise, in the following order, anion exchange chromatography, hydrophobic interaction chromatography, and cation exchange chromatography. In a specific embodiment, the one or more steps of column chromatography comprise, in the following order, cation exchange chromatography, anion exchange chromatography, and hydrophobic interaction chromatography. In a specific embodiment, the one or more steps of column chromatography comprise, in the following order, cation exchange chromatography, hydrophobic interaction chromatography, and anion exchange chromatography.

In various aspects and embodiments, the 900 kDa BoNT/A complex is produced by a process that does not comprise a step of precipitation with cold ethanol, hydrochloric acid, or ammonia sulfate during purification of the 900 kDa BoNT/A complex. In specific embodiments, the 900 kDa BoNT/A complex is produced by a process that does not comprise a step of precipitation with cold ethanol. In specific embodiments, the 900 kDa BoNT/A complex is produced by a process that does not comprise a step of precipitation with hydrochloric acid. In specific embodiments, the 900 kDa BoNT/A complex is produced by a process that does not comprise a step of precipitation with ammonia sulfate.

In various aspects and embodiments, the 900 kDa BoNT/A complex is produced by a process that comprises the following steps: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, and filtering at 0.2 μm with added ammonium sulfate.

In various aspects and embodiments, the 900 kDa BoNT/A complex is produced by a process that comprises one or more of the following steps: subjecting fermentation culture to acid precipitation using 3M sulfuric acid to reduce pH to 3.5 at a temperature below 25°C, subjecting the acid precipitate to tangential flow filtration (*e.g.*, 0.1 μm tangential flow filtration) to concentrate cell mass, adjusting pH to about 6.0, adding one or more nucleases to reduce host cell nucleic acid content, clarifying by centrifugation to remove cell debris, and filtering at 0.2 μm with added ammonium sulfate.

In various aspects and embodiments, the 900 kDa BoNT/A complex is produced by a process that comprises the following steps and in the following order: subjecting fermentation culture to acid precipitation using 3M sulfuric acid to reduce pH to 3.5 at a temperature below 25°C, subjecting the acid precipitate to tangential flow filtration (*e.g.*, 0.1 μm tangential flow filtration) to concentrate cell mass, adjusting pH to about 6.0, adding one or more nucleases to reduce host cell nucleic acid content, clarifying by centrifugation to remove cell debris, and filtering at 0.2 μm with added ammonium sulfate.

In various aspects and embodiments, the 900 kDa BoNT/A complex is produced by a process that comprises the following steps: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, filtering at 0.2 μm with added ammonium sulfate, and subjecting the filtrate to hydrophobic interaction chromatography (optionally followed by anion exchange chromatography and cation exchange chromatography).

In various aspects and embodiments, the 900 kDa BoNT/A complex is produced by a process that comprises one or more of the following steps: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, filtering at 0.2 μm with added ammonium sulfate, and subjecting the filtrate to hydrophobic interaction chromatography (optionally followed by anion exchange chromatography and cation exchange chromatography).

In various aspects and embodiments, the 900 kDa BoNT/A complex is produced by a process that comprises the following steps and in the following order: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, filtering at 0.2 μm with added ammonium sulfate, and subjecting the filtrate to hydrophobic interaction chromatography (optionally followed by anion exchange chromatography and cation exchange chromatography).

In various aspects and embodiments, the 900 kDa BoNT/A complex is produced by a process that comprises the following steps: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, filtering at 0.2 μm with added ammonium sulfate, loading the filtrate onto a hydrophobic interaction column, eluting with a descending gradient of ammonium sulfate, and isolating the product peak (optionally followed by anion exchange chromatography and cation exchange chromatography).

In various aspects and embodiments, the 900 kDa BoNT/A complex is produced by a process that comprises one or more of the following steps: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, filtering at 0.2 μm with added ammonium sulfate, loading the filtrate onto a hydrophobic interaction column, eluting with a descending gradient of ammonium sulfate, and isolating the product peak (optionally followed by anion exchange chromatography and cation exchange chromatography).

In various aspects and embodiments, the 900 kDa BoNT/A complex is produced by a process that comprises the following steps and in the following order: subjecting fermentation culture to acid precipitation using 3M sulfuric acid to reduce pH to 3.5 at a temperature below 25°C, subjecting the acid precipitate to tangential flow filtration (*e.g.,* 0.1 μm tangential flow filtration) to concentrate cell mass, adjusting pH to about 6.0, adding one or more nucleases to reduce host cell nucleic acid content, clarifying by centrifugation to remove cell debris, filtering at 0.2 μm with added ammonium sulfate, loading the filtrate onto a hydrophobic interaction column, eluting with a descending gradient of ammonium sulfate, and isolating the product peak (optionally followed by anion exchange chromatography and cation exchange chromatography).

In certain embodiments, the 900 kDa BoNT/A complex is produced by *Clostridium botulinum* bacteria cultured and expanded from an animal product free working cell bank.

In various aspects and embodiments, the 900 kDa BoNT/A complex is produced by a process that does not involve using a protease inhibitor. In certain embodiments, the 900 kDa BoNT/A complex is produced by a process that does not involve using benzamidine hydrocholoride.

After stabilization in a suitable solution, the bulk BoNT/A drug substance can be compounded with one or more excipients (*e.g.,* human serum albumin, such as recombinant human serum albumin, and sodium chloride) and can then be sterile filtered to make a pharmaceutical composition suitable for administration to a human. An example of such a BoNT/A is onabotulinumtoxinA. The pharmaceutical compositions can be made into a solid form (*e.g.,* as powder) by drying (*e.g.,* vacuum-drying or freeze-drying). Solid pharmaceutical compositions can be stored and reconstituted prior to injection. The pharmaceutical composition can also be in a liquid form (*e.g.,* as a solution). Liquid pharmaceutical compositions can be stored and used directly for injection. The BoNT/A pharmaceutical compositions described herein can comprise a 900 kDa *Clostridium botulinum* neurotoxin serotype A (BoNT/A) complex as an active pharmaceutical ingredient. The pharmaceutical composition can also include one or more excipients, buffers, carriers, stabilizers, preservatives and/or bulking agents. Such pharmaceutical compositions are preferably chemically and physically stable such that the BoNT/A active pharmaceutical ingredient remains suitable for use as a pharmaceutical product following storage. BoNT/A products may be stored at room temperature, in refrigerated conditions, or below 0°C. It is preferable that the BoNT/A remains stable during storage for at least about 12 months, more preferably at least about 18 months.

In various aspects and embodiments, the composition described herein is animal product free. In various aspects and embodiments, the composition described herein does not contain a protease inhibitor. In certain embodiments, the composition described herein does not contain benzamidine hydrocholoride. In various aspects and embodiments, the composition described herein is produced by a process that does not involve using a protease inhibitor. In certain embodiments, the composition described herein is produced by a process that does not involve using benzamidine hydrocholoride. In various aspects and embodiments, the 900 kDa BoNT/A complex is produced by *Clostridium botulinum* bacteria cultured and expanded from an animal product free working cell bank. The animal product free working cell bank can be produced, for example and without limitation, as described in Example 6.

In some embodiments, an animal product free composition is free of human derived human serum albumin (HSA). In some embodiments, an animal product free composition is free of animal derived nucleases. In some embodiments, an animal product free composition comprises recombinantly produced HSA. In some embodiments, the recombinantly produced HSA is commercially available or known in the art. In some embodiments, an animal product free composition is ammonium sulfate free. In some embodiments, an animal product free composition comprises chromatography resin.

In various aspects and embodiments, the composition described herein further comprises human serum albumin (HSA). In certain embodiments, the composition comprises about 0.5 mg of HSA per 100 units of the 900 kDa BoNT/A complex. In certain embodiments, the HSA is recombinant HSA. In specific embodiments, the recombinant HSA is animal product free. In specific embodiments, the recombinant HSA is not produced from an animal. In specific embodiments, the recombinant HSA is produced from a microorganism, such as bacteria. In specific embodiments, the recombinant HSA is produced from a plant-based expression system. In certain embodiments, the HSA is human plasma-derived.

In various aspects and embodiments, the composition described herein further comprises sodium chloride. In certain embodiments, the composition comprises about 0.9 mg of sodium chloride per 100 units of the 900 kDa BoNT/A complex.

In various aspects and embodiments, the composition described herein comprises about 50 units of the 900 kDa BoNT/A complex. In various aspects and embodiments, the composition described herein comprises about 100 units of the 900 kDa BoNT/A complex. In various aspects and embodiments, the composition described herein comprises about 200 units of the 900 kDa BoNT/A complex.

In various aspects and embodiments, the composition described herein is a liquid composition. In various aspects and embodiments, the composition described herein is a solid composition. In certain embodiments, the composition described herein is a vacuum-dried composition. In certain embodiments, the composition described herein is a freeze-dried composition. In certain embodiments, the composition described herein is a powdered pharmaceutical composition. In specific embodiments, the composition described herein is a drug product. In a specific embodiment, the composition described herein is a drug product and further comprises HSA (*e.g.,* about 0.5 mg of HSA per 100 units of the 900 kDa BoNT/A complex) and sodium chloride (*e.g.*, about 0.9 mg of sodium chloride per 100 units of the 900 kDa BoNT/A complex). In specific embodiments, the composition described herein is a drug substance. In a specific embodiment, the composition described herein is a drug substance in a solution suitable for storage.

In one aspect, provided herein is a BoNT/A composition produced by a method or process described in this Section 5.4.

## 5.5. Characterization of BoNT/A Compositions

In one embodiment, BoNT/A compositions described herein have a potency of at least about $1.5 \times 10^7$ units/mg, or at least about $2.0 \times 10^7$ units/mg, *e.g.*, about $1.5 \times 10^7$ to about $6.0 \times 10^7$ units/mg, about $2.0 \times 10^7$ to about $6.0 \times 10^7$ units/mg, about $2.4 \times 10^7$ to about $6.0^x 10^7$ units/mg, about $2.4 \times 10^7$ to about $5.9 \times 10^7$ units/mg, about $2.4 \times 10^7$ to about $5.8 \times 10^7$ units/mg, about $2.4 \times 10^7$ to about $5.7 \times 10^7$ units/mg, about $2.4 \times 10^7$ to about $5.6 \times 10^7$ units/mg, about $2.4 \times 10^7$ to about $5.5 \times 10^7$ units/mg, about $2.4 \times 10^7$ to about $5.4 \times 10^7$ units/mg, about $2.5 \times 10^7$ to about $6.0^x 10^7$ units/mg, about $2.6 \times 10^7$ to about $6.0^x 10^7$ units/mg, about $2.7 \times 10^7$ to about $6.0 \times 10^7$ units/mg, about $2.8 \times 10^7$ to about $6.0^x 10^7$ units/mg, about $2.9 \times 10^7$ to about $6.0 \times 10^7$ units/mg, about $3.0 \times 10^7$ to about $6.0 \times 10^7$ units/mg, or any numbers between such ranges.

In one embodiment, BoNT/A compositions described herein have a potency of about $2.4 \times 10^7$ units/mg to about $5.4 \times 10^7$ units/mg. In preferred embodiments, the term "unit" used herein refers to the LD50 dose.

In various embodiments and aspects, a BoNT/A composition described herein has a higher potency than a commercially available BoNT/A composition that is known in the art. In certain embodiments, a BoNT/A composition described herein has a potency that is at least 10%, at least 20%, at least 30%, at least 40%, at least 50%, at least 60%, at least 70%, at least 80%, at least 90%, at least 100%, at least 1.5-fold, or at least 2-fold higher than the potency of a commercially available BoNT/A composition that is known in the art. In certain embodiments, a BoNT/A composition described herein has a potency that is at least $0.1 \times 10^7$ units/mg, at least $0.2 \times 10^7$ units/mg, at least $0.3 \times 10^7$ units/mg, at least $0.4 \times 10^7$ units/mg, at least $0.5 \times 10^7$ units/mg, at least $0.6 \times 10^7$ units/mg, at least $0.7 \times 10^7$ units/mg, at least $0.8 \times 10^7$ units/mg, at least $0.9 \times 10^7$ units/mg, at least $1 \times 10^7$ units/mg, at least $1.5 \times 10^7$ units/mg, at least $2 \times 10^7$ units/mg, at least $3 \times 10^7$ units/mg, or at least $4 \times 10^7$ units/mg higher than the potency of a commercially available BoNT/A composition that is known in the art.

The potency of the BoNT/A compositions described herein can be determined with methods known in the art, including but not limited to, *e.g.,* Light-Chain Activity High-Performance Liquid Chromatography (LCA-HPLC) assay, mouse 50% lethal dose (MLD$_{50}$) assay, Mouse Digit Abduction Score (DAS) assay, SNAP-25 assay, cell-based potency assay (CBPA), etc.

The LCA-HPLC assay measures SNAP-25 cleavage specificity. Samples are reacted with a commercially available BoNT/A fluorescent substrate derived from the SNAP-25 sequence. The fluorescently-labeled cleavage products are separated and detected via a reverse-phase (RP)-HPLC method. Further description of LCA-HPLC assay can be found in publications Hunt et al. (2010) Toxins 2(8):2198-2212 and Rupp et al. (2020) Toxins 12(6):393, each of which is incorporated herein by reference in its entirety.

In one embodiment, the potency is determined using a mouse 50% lethal dose (MLD50) assay. The mouse 50% lethal dose (MLD50) assay has been described in, *e.g.*, Schantz and Kautter (1978) Journal of the AOAC 61(1):96-99, Hunt and Kenneth (2009) Clinical Neuropharmacology 32(1):28-31, U.S. Patent No. 7,160,699, and U.S. Patent No. 9,725,705, each of which is incorporated herein by reference in its entirety. Mouse 50% lethal dose (MLD50) assay is a method for measuring the potency of a botulinum toxin by intraperitoneal injection of the botulinum toxin into female mice (about four weeks old) weighing 17-22 grams each at the start of the assay. Each mouse is held in a supine position with its head tilted down and is injected intraperitoneally into the lower right abdomen at an angle of about 30 degrees using a 25 to 27 gauge 3/8" to 5/8" needle with one of several serial dilutions of the botulinum toxin in saline. The death rates over the ensuing 72 hours for each dilution are recorded. The dilutions are prepared so that the most concentrated dilution produces a death rate of at least 80% of the mice injected, and the least concentration dilution produces a death rate of no greater than 20% of the mice injected. There must be a minimum of four dilutions that fall within the monotone decreasing range of the death rates. The monotone decreasing range commences with a death rate of no less than 80%. Within the four or more monotone decreasing rates, the two largest and the two smallest rates must be decreasing (*i.e.,* not equivalent). The dilution at which 50% of the mice die within the three day post injection observation period is defined as a dilution which comprises one unit (1 U) of the botulinum toxin.

Mouse Digit Abduction Score (DAS) assay is an in vivo assessment of toxin-induced muscle paralysis following injection of BoNT/A toxin into the hind limb muscle of a rodent. The DAS assay can be used to assess the potency of BoNT/A compositions on muscle paralysis, as well as the duration of action. Detailed protocols of DAS assay have been disclosed in Aoki et al. (1999) Eur. J. Neurol. 6:s3-s10, Aoki (2001) Toxicon 39: 1815-1820, Broide et al. (2013) Toxicon 71:18-24, and Rupp et al. (2020) Toxins 12(6):393, each of which is incorporated herein by reference in its entirety. For example, the DAS assay can be performed by injection of a BoNT/A composition described herein into the mouse gastrocnemius/soleus complex, followed by assessment of Digital Abduction Score using the method of Aoki (2001) Toxicon 39:1815-1820. In the DAS assay, mice are suspended briefly by the tail in order to elicit a characteristic startle response in which the mouse extends its hind limbs and abducts its hind digits. Following the BoNT/A composition injection, the varying degrees of digit abduction are scored on a five-point scale (0=normal to 4=maximal reduction in digit abduction and leg extension). Safety Ratio, the ratio between the amount of a toxin required for a 10% drop in a bodyweight (measured at peak effect within the first seven days after dosing in a mouse) and the amount of toxin required for a DAS score of 2, can also be determined to assess the therapeutic index of the BoNT/A composition described herein, as described in U.S. Patent. No. 9,920,310, which is incorporated by reference herein in its entirety. High Safety Ratio scores are therefore desired, and indicate a toxin that is able to effectively paralyze a target muscle with little undesired off-target effects.

SNAP-25 assay is an ELISA based method to measure SNAP-25 proteolytic activity of the botulinum toxin. The assay uses a truncated SNAP-25 protein (the 206 amino acid residue peptide) bound to polystyrene 96 well microtiter plates and a monoclonal antibody that recognizes the cleaved product (a 197 amino acid residue peptide) which is made by enzymatic hydrolysis between amino acids 197 and 198 of the SNAP-25 by reduced botulinum toxin type A. The monoclonal antibody bound to the cleaved product is then detected with a secondary antibody (goat anti-mouse IgG conjugated to horseradish peroxidase HRP), which produces a color change in the presence of a chromogenic substrate (TMB). Exemplary SNAP-25 methods are described in Ekong et al. (1997) Microbiology 143:3337-3347, and U.S. Patent No. 7,160,699, each of which is incorporated herein by reference.

Cell-based potency assay (CBPA) has been described in *e.g.,* Fernández-Salas et al. (2012) PLOS ONE 7(11):e49516, Rupp et al. (2020) Toxins 12(6):393, WO 2010/105234, and WO 2009/114748, each of which is incorporated herein by reference. In one embodiment, the SNAP-25$_{197}$ SiMa H1 electrochemiluminescent (ECL) CBPA is used to determine the potency of the BoNT/A compositions described herein. The SNAP-25$_{197}$ SiMa H1 electrochemiluminescent (ECL) CBPA is an in vitro cell-based assay that measures the key steps of BoNT/A intoxication: receptor-mediated cell binding and internalization, translocation of the protease domain (light chain) into the cytosol, and proteolytic cleavage of SNAP-25, allowing direct comparison of BoNT/A product biological activity in vitro (Fernandez-Salas et al. (2012) PLOS ONE 7(11):e49516; Rupp et al. (2020) Toxins 12(6):393). In brief, human neuroblastoma SiMa H1 cells are plated onto poly-D-lysine (PDL) 96-well plates in serum-free media (SFM) with 25 $\mu$g/mL of $GT_{1b}$ for three days and treated with toxin samples for 24 hours. After treatment, toxins are removed, cells are lysed and lysates are transferred to MSD High Bind plates coated with anti-SNAP-25$_{197}$ monoclonal antibody (mAb) 2E2A6. Plates are then washed and incubated with SULFO-TAG NHS-Ester labeled anti-SNAP-25 polyclonal antibody (pAb) for detection. Captured, BoNT/A toxin-cleaved SNAP-25 is then quantitated on a MSD plate reader.

## 5.6. Illustrative Embodiments

The present disclosure includes the following non-limiting illustrative embodiments.

1. A composition comprising a plurality of 900 kDa *Clostridium botulinum* serotype A (BoNT/A) neurotoxin complex species, wherein the oxidation level of 150 kDa neurotoxin species present in the composition is less than 6% at each of the following positions: 411 (M411) as shown in SEQ ID NO:2, 550 (M550) as show in SEQ ID NO:3, 1004 (M1004) as shown in SEQ ID NO:3, and 1144 (M1144) as shown in SEQ ID NO:3.

2. The composition of embodiment 1, wherein the 900 kDa BoNT/A complex is onabotulinumtoxinA.

3. The composition of embodiment 1 or 2, wherein the oxidation level at position M411 of SEQ ID NO. 2 is less than 5%.

4. The composition of any one of embodiments 1-3, wherein the oxidation level at position M411 of SEQ ID NO. 2 is about 2%.

5. The composition of any one of embodiments 1-4, wherein the oxidation level at position M550 of SEQ ID NO. 3 is less than 1%.

6. The composition of any one of embodiments 1-5, wherein the oxidation level at position M550 of SEQ ID NO. 3 is about 0.3% to about 0.5%.

7. The composition of any one of embodiments 1-6, wherein the oxidation level at position M1004 of SEQ ID NO. 3 is less than 3%.

8. The composition of any one of embodiments 1-7, wherein the oxidation level at position M1004 of SEQ ID NO. 3 is about 1% to about 2%.

9. The composition of any one of embodiments 1-8, wherein the oxidation level at position M1144 of SEQ ID NO. 3 is less than 6%.

10. The composition of any one of embodiments 1-9, wherein the oxidation level at position M1144 of SEQ ID NO. 3 is about 2%.

11. The composition of any one of embodiments 1-10, wherein the oxidation level is determined by LC-MS/MS.

12. The composition of any one of embodiments 1-11, wherein the composition comprises less than 3% of host cell protein.

13. The composition of any one of embodiments 1-12, wherein the composition has a potency of about 1.5 x 10$^7$ units/mg to about 6.0 x 10$^7$ units/mg.

14. The composition of embodiment 13, wherein the potency is determined using a mouse 50% lethal dose (MLD50) assay.

15. The composition of embodiment 13, wherein the potency is determined using a cell based potency assay.

16. The composition of any one of embodiments 1-15 further comprising a pharmaceutically acceptable carrier.

17. The composition of embodiment 16, wherein the pharmaceutically acceptable carrier comprises human serum albumin and sodium chloride.

18. The composition of any one of embodiments 1-17, wherein the composition is vacuum-dried.

19. The composition of any one of embodiments 1-18, wherein the 900 kDa BoNT/A complex is produced in a fermentation condition comprising a cold shock fermentation temperature.

20. The composition of any one of embodiments 1-18, wherein the 900 kDa BoNT/A complex is produced in a continuous cold temperature fermentation condition.

21. The composition of any one of embodiments 1-20, wherein the 900 kDa BoNT/A complex is purified by one or more steps of column chromatography.

22. The composition of embodiment 21, wherein the one or more steps of column chromatography comprise hydrophobic interaction chromatography.

23. The composition of embodiment 22, wherein the one or more steps of column chromatography further comprise anion exchange chromatography.

24. The composition of embodiment 22 or 23, wherein the one or more steps of column chromatography further comprise cation exchange chromatography.

25. The composition of any one of embodiments 1-24, wherein the 900 kDa BoNT/A complex is produced by *Clostridium botulinum* bacteria cultured and expanded from an animal product free working cell bank.

26. The composition of any one of embodiments 1-25, which is animal product free.

27. The composition of any one of embodiments 1-26, which does not contain a protease inhibitor.

28. The composition of any one of embodiments 1-27, which does not contain benzamidine hydrochloride.

29. A method of producing a composition comprising BoNT/A, said method comprising incubating a culture of *Clostridium botulinum* bacteria in a production fermentor at a temperature that is below 35 °C for a period of time.

30. The method of embodiment 29, wherein the entire fermentation period is about 72 hours.

31. The method of embodiment 29, wherein the entire fermentation period is about 160 hours.

32. The method of any one of embodiments 29-31, wherein the period of time is about 5 hours.

33. The method of any one of embodiments 29-31, wherein the period of time is the entire fermentation period.

34. The method of any one of embodiments 29-33, wherein the temperature is about 15 °C.

35. The method of any one of embodiments 29-33, wherein the temperature is about 20 °C.

36. The method of any one of embodiments 29-33, wherein the temperature is about 25 °C.

37. The method of embodiment 29 or 30, which comprises, in the following order: (a) incubating the culture of *Clostridium botulinum* bacteria in the production fermentor at 35 °C for about 12 hours; (b) setting temperature to 15 °C at the end of step (a); (c) culturing at 15 °C until about 5 hours after step (a); (d) setting temperature to 35 °C at the end of step (c); and (e) culturing at 35 °C until about 55 hours after step (c).

38. The method of embodiment 29 or 30, which comprises, in the following order: (a) incubating the culture of *Clostridium botulinum* bacteria in the production fermentor at 35 °C for about 12 hours; (b) setting temperature to 20 °C at the end of step (a); (c) culturing at 20 °C until about 5 hours after step (a); (d) setting temperature to 35 °C at the end of step (c); and (e) culturing at 35 °C until about 55 hours after step (c).

39. The method of embodiment 29 or 31, which comprises incubating the culture of Clostridium botulinum bacteria in the production fermentor at 25 °C for about 160 hours

40. A method of treating a patient in need thereof, comprising administering a composition according to any one of embodiments 1-28.

41. A method substantially as described herein.

42. A composition substantially as described herein.

## 6. EXAMPLES

Certain embodiments provided herein are illustrated by the following non-limiting examples, which describe different methods for obtaining BoNT/A and demonstrate that lowering the temperature for a short period of time or continuously during fermentation of *Clostridium botulinum* reduced the oxidation levels at one or more of the methionine residues (*e.g.,* M411, M550, M1004, and/or M1144) of the produced 150 kDa neurotoxin component of BoNT/A and that there is a negative correlation between the oxidation level of BoNT/A and its potency.

### 6.1. Example 1: Production of BoNT/A Complex under Various Fermentation Temperatures

1. Upstream Steps

There were two general stages to generate BoNT/A complex material prior to analysis of attributes: an Upstream stage and a Downstream stage. The Upstream stage included using a 50 mL APF seed culture medium in a 150 mL glass bottle (with a magnetic stir bar) containing 3.25 % w/v soy peptone type II, 1.2 % w/v yeast extract, 1.5 % w/v glucose, pH adjusted to 7.3 using sodium hydroxide. The seed culture medium was reduced to remove oxygen (in a Don Whitley Scientific A55 anaerobic chamber) for a minimum of 48 hours prior to use. The seed culture medium was inoculated with 50 $\mu$L of a thawed *C. botulinum* serotype A Hall strain working cell bank. The seed culture was incubated at 35 $\pm$ 1°C with 150 rpm stirring in the anaerobic chamber.

After overnight seed culture incubation, 4 mL of culture ($OD_{600}$ nm 4.81 $\pm$ 0.35) was transferred to a production fermentor (Ambr® 250 Modular, Sartorius Stedim) containing 196 mL of reduced APF fermentation medium (3.25 % w/v soy peptone type II, 1.2 % w/v yeast extract, 1.5 % w/v glucose, pH adjusted to 7.3 using sodium hydroxide). The temperature for each fermentation was controlled according to Table 2. Temperature adjustments for cold-shock fermentations were started at the early stationary phase of cell growth. Agitation was controlled at 150 rpm and the fermentor headspace was overlaid with $N_2$ at 50 mL/min to maintain an anaerobic environment. Fermentation pH and cell growth were monitored by an online pH probe and reflectance monitor, respectively. The three phases for the production fermentation include exponential growth, stationary, and autolysis phases. The fermentation times were varied from 72 hrs to 160 hrs (Table 2) to ensure cell lysis occurred, which released active BoNT/A into the culture medium.

Table 2

| Experimental Description | Production Fermentation* Condition | Production Fermentation Time (hours) |
|---|---|---|
| Baseline Control Fermentation | 35 °C throughout fermentation period | 72 |
| 20 °C Cold-shock | 35 °C from 0 to 12 hrs; temperature set to 20 °C at 12 hrs; culture at 20 °C until 17 hrs, then temperature set to 35 °C at 17 hrs; culture at 35 °C until 72 hrs | 72 |
| 15 °C Cold-shock | 35 °C from 0 to 12 hrs; temperature set to 15 °C at 12 hrs; culture at 15 °C until 17 hrs, then temperature set to 35 °C at 17 hrs; culture at 35 °C until 72 hrs. | 72 |
| 25 °C Fermentation | 25 °C throughout fermentation period | 160 |
| * The Ambr® 250 Modular system can control temperature to within ± 0.5 °C. | | |

The harvest step of BoNT/A complex utilized centrifugation followed by 0.2 μm filtration to remove cellular debris from the culture broth, which contained BoNT/A complex and other host-cell proteins. Cellular debris was initially removed by centrifugation at 10,000 rcf for 30 minutes. The clarified supernatant was then subjected to 0.2 μm filtration in a biological safety cabinet to ensure any remaining *C. botulinum* type A organism and cellular debris was removed. Complete removal of organism was confirmed (by plating a portion of the 0.2 μm filtrate on reduced Columbia Blood Agar plates and incubating under anaerobic conditions for a sufficient time to verify absence of viable *C. botulinum* type A) and the clarified harvest material was stored at 4 °C until start of the Downstream stage.

2. Downstream Steps

Downstream steps included a concentration and buffer exchange using tangential flow filtration (TFF), followed by a capture of the botulinum neurotoxin on an anion exchange column, elution from the column and further separation from impurities by polishing on a cation exchange column.

A tangential flow filtration system was used to concentrate (by ultrafiltration - UF) and diafilter the clarified fermentation harvest material into 50 mM sodium phosphate, pH 6.5 buffer. Repligen TangenX® SIUS PD cassettes with a 100 kDa molecular weight cut off membrane were used for the concentration and diafiltration steps. The UF/DF step concentrated the clarified harvest material 3 to 4-fold and then diafiltered using 50 mM sodium phosphate, pH 6.5 buffer.

Particulars of the ultrafiltration/diafiltration (UF/DF) process used were as follows. The UF/DF unit and Repligen 100 kDa membrane with $0.02\ m^2$ membrane area was initially flushed with a minimum of 0.2 L of water for injection (WFI) to remove the membrane storage solution. Next, the membrane and UF/DF system were equilibrated with approximately 0.2 L of 50 mM sodium phosphate, pH 6.5 buffer. After membrane equilibration, the clarified fermentation harvest material was loaded onto the TangenX® tangential flow filtration cassette and concentrated 3 to 4-fold at a transmembrane pressure range of 5 to 6 psig (pounds per square inch gauge) and flow rate set at 75 mL/min. Following the concentration step, the retentate pool was diafiltered against a minimum of 5 diafiltration volumes of the 50 mM sodium phosphate, pH 6.5 buffer at a transmembrane pressure range of 5 to 8 psig and flow rate set at 75 mL/min. After completion of diafiltration, the pressure was released from the retentate line and the permeate outlet was closed. The UF/DF material was then recirculated for at least 5 minutes and the system was then drained followed by rinsing with 50 mM sodium phosphate, pH 6.5 buffer as the UF/DF material was recovered into a separate container.

The recovered material (UF/DF retentate) from the UF/DF step was then loaded onto an anion exchange chromatography column packed with POROS® 50HQ resin. The column has an inner diameter of 1.13 cm and a column height of about 5 cm. The entire anion exchange column chromatography was performed at ambient temperature, and the flow was in the downward direction. The botulinum neurotoxin type A complex was eluted from the anion exchange column using a pH step change where the more negatively charged impurities such as nucleic acids (*e.g.* DNA and RNA) and other host cell proteins remained bound to the anion exchange column.

Particulars of the anion exchange step were: use of the POROS® 50HQ column equilibrated with a 50 mM sodium phosphate, pH 6.5 buffer (at least 5 column volumes). Next the UF/DF retentate was loaded at 120 cm/hour onto the POROS® 50HQ anion exchange column, followed by washing with at least about 10 column volumes of 50 mM sodium phosphate, pH 6.5 at 120 cm/hour, followed by eluting with 50 mM sodium acetate, pH 4.5 at 120 cm/hour. The peak fraction was collected, when the absorbance at 280 nm (A280) increased to at least about 0.05 AU and through the peak maximum to equal or less than about 0.03 AU on the trailing edge, into a container with 5 mL of 50 mM sodium acetate, pH 4.8. This elution pool was stored at about 2° C. to about 8° C. for up to 48 hours.

The second chromatography step in the downstream process used a POROS® 20HS cation exchange chromatography resin packed into a column with an inner diameter of 0.46 cm and a column height of 10.0 cm. The entire POROS® 20HS column chromatography was performed at ambient temperature, and the flow was in the downward direction. The botulinum neurotoxin type A complex binds to the POROS® 20HS column at pH 4.8. After wash steps to remove impurities, the bound botulinum neurotoxin type A complex was then eluted from the column with increased NaCl concentration. The product-related impurities were eluted with the wash buffer and decontamination solution.

Particulars of the cation exchange step were: use of the POROS® 20HS column equilibrated with a 50 mM sodium acetate, pH 4.8 buffer (at least about 5 column volumes). Next the POROS® 50HQ product pool (collected as described above, fresh or from storage at 2 - 8°C) was loaded onto the POROS® 20HS column. The column was then washed with a 50 mM sodium acetate, pH 4.8 buffer (at least about 5 column volumes) and then washed again with 10 column volumes of second buffer (50 mM sodium acetate, 80 mM sodium chloride, pH 4.8 buffer). The botulinum neurotoxin type A complex was eluted from the POROS® 20HS column with a 50 mM sodium acetate, 350 mM sodium chloride, pH 4.8 buffer at 290 cm/hour. The eluate was collected into container with 3 mL of 50 mM sodium acetate, pH 4.8 when the A280 increased to about ≥0.02 AU through peak maximum until the A280 of the trailing edge of the elution peak decreased to a value of ≤0.02 AU or until end of the elution step, which had a total of 5 column volumes starting from the end of the second wash step. The POROS® 20HS product pool was stored for the short term at about 2°C to 8°C for up to 120 hours. The product pool was next aliquoted into small volume samples and stored for longer term in a freezer at -70°C prior to analysis.

## 6.2. Example 2: LC-MS/MS Analysis of Samples from Temperature Studies

Samples were denatured by heating at 90±5 °C in the presence of 0.05% RapiGest™, reduced with 5 mM dithiothreitol and alkylated with 15 mM iodoacetamide. Excess iodoacetamine was removed by Zeba spin columns. Then the samples were digested with a ratio of 1:10 trypsin for 4+1 hours at 37+3 °C. RapiGest™ was removed by acidification and subsequent centrifugation at 20,000 rcf.

The resulting peptides were analyzed using Thermo Vanquish UHPLC coupled to a Thermo Q Exactive Plus mass spectrometer. Five picomoles of each sample digest were injected for LC-MS/MS analysis. Mass spectrometry of the peptides eluting from the reversed phase column was performed using electrospray ionization in positive mode. The LC-MS/MS parameters used for the analysis of the digest samples are provided in Table 3.

Table 3 LC-MS Experimental Conditions

| Sample | Trypsin digests of drug substance | | | |
|---|---|---|---|---|
| Sample load | 5 picomoles | | | |
| Instrument | Thermo Vanquish UHPLC and Thermo Q Exactive Plus | | | |
| Column | Waters BEH C18 2.1 mm x 150 mm 1.7 $\mu$m | | | |
| Mobile Phase A (MPA) | 0.1% formic acid in water | | | |
| Mobile Phase B (MPB) | 0.1% formic acid in 100% acetonitrile | | | |
| Gradient | Time (min) | Flow rate (ml/min) | % MPA | % MPB |
| | 0 | 0.2 | 99 | 1 |
| | 7 | 0.2 | 99 | 1 |
| | 7.1 | 0.2 | 95 | 5 |
| | 60 | 0.2 | 65 | 35 |
| | 70 | 0.2 | 20 | 80 |
| | 70.1 | 0.2 | 99 | 1 |
| | 75.0 | 0.2 | 99 | 1 |

The Q Exactive Plus (.raw) data files were searched using Thermo BiopharmaFinder. The search parameters used for the database analysis are provided in **Error! Reference source not found**.4.

Table 4 BiophannaFinder Search Parameters

| Parameter | Value |
|---|---|
| Enzyme | trypsin |
| Max. Missed Cleavages | 1 |
| Fixed Modifications | none |
| Variable Modifications | Carbamidomethylation (C), Deamidated (N), Dioxidation (C), Oxidation (MW) |
| Peptide Tolerance | $\pm$ 8 ppm |
| MS/MS Ions Search | yes |

Oxidation levels at various methionine residues of BoNT/A are shown in Table 5 below:

Table 5

| | Baseline Control Fermentation | 20 °C Cold-shock | 15 °C Cold-shock | 25 °C Fermentation |
|---|---|---|---|---|
| M30 | 0.3 | 0.2 | 0.3 | 0.3 |
| M106 | 1 | 1 | 1 | 1 |
| M344 | 1 | 1 | 1 | 1 |
| M411 | 5 | 2 | 2 | 2 |
| M550 | 1 | 0.3 | 0.4 | 0.5 |
| M1004 | 3 | 1 | 1 | 2 |
| M1054 | 0.5 | 0.8 | 0.6 | 0.6 |
| M1144 | 6 | 2 | 2 | 2 |

Cold fermentation conditions reduced oxidation levels at M411, M550, M1004, and M1144 of BoNT/A (Table 5). M411 is part of the light chain and all other methionine residues (M550, M1004, and M1144) reside in the heavy chain. The crystal structure of the BoNT/A toxin receptor-binding domain in complex with the SV2C receptor has been solved (Benoit et al. (2017) Sci Rep. 7:43588). It is shown that the interaction includes amino acids Thr1145, Thr1146 and Ser1294 on BoNT/A (Benoit et al. (2017) Sci Rep. 7:43588). Since M1144 is adjacent to these binding residues (Thr1145 and Thr1146) of BoNT/A with the SV2C receptor, its oxidation can cause a decrease in receptor binding and thus, loss of potency.

### 6.3. Example 3: SEC-HPLC Analysis of Samples from Temperature Studies

Samples were equilibrated at room temperature for at least 20 minutes. 100 $\mu$L of each sample was transferred to a 0.3 mL polypropylene HPLC vial for direct injection. The SEC-HPLC analysis was performed using a Waters® HPLC with UV detector following the instrument conditions provided in Table 6.

Table 6 SEC-HPLC Experimental Conditions

| | |
|---|---|
| Column | Sepax SRT SEC-500, 7.8x300mm, 5-micron (PN 215500-7830) |
| Column temperature | 30 °C |
| Mobile phase | 40 mM Sodium Phosphate + 200 mM Ammonium Sulfate, pH 6.5 |
| Flow rate | 0.8 mL/min |
| UV Detection | 220 nm |
| Injection volume | Adjust injection volume based on UV concentration to inject 20 $\mu$g |
| Auto sampler temperature | 5 °C |
| Run time | 20 minutes |

The chromatography data were processed with Waters® Empower software. The higher molecular weight species elute early than the lower ones. All peaks not present in the buffer analysis were integrated. The relative peak area of each peak was reported.

SEC data (%Area) at UV220nm are shown below in Table 7.

Table 7

| | | HCP (<100KDa) |
|---|---|---|
| Baseline Control Fermentation | AEC | 15.2 |
| 20 °C Cold-shock | | 0.0 |
| 15 °C Cold-shock | | 1.5 |

| 25 °C Fermentation | CEC | 31.3 |
|---|---|---|
| Baseline Control Fermentation | | 0.0 |
| 20 °C Cold-shock | | 0.0 |
| 15 °C Cold-shock | | 0.0 |
| 25 °C Fermentation | | 3.6 |

Table 7 shows that the percentage of the host cell protein (HCP) was reduced in both 20°C and 15°C cold shock conditions compared to the baseline control fermentation condition after the single anion exchange column (AEC) purification, whereas 25°C continuous cold fermentation condition had a higher percentage of HCP compared to the baseline control. After the two-column (*i.e.,* AEC followed by CEC) purification process, HCP in the 25°C continuous cold fermentation condition was higher than 3% while it was undetectable in both the Baseline Control and the Cold-shock conditions.

### 6.4. Example 4: Characterization of Oxidation Sites of 150kDa BoNT/A and Related Effects on Potency

*Executive Summary*

The oxidation pathway and potency of *Clostridium botulinum* type A neurotoxin (BoNT/A) complex after exposure to hydrogen peroxide ($H_2O_2$) was characterized. Various amounts of hydrogen peroxide ($H_2O_2$) ranging from 100 ppb to 100 ppm were added to the BoNT/A drug substance (DS) and the resulting oxidation sites on the 150 kDa neurotoxin (150 kDa NT) subunit were identified and quantified by liquid chromatography coupled with tandem mass spectrometry (LC-MS/MS). The results showed that several methionine residues on the 150 kDa NT are susceptible to oxidation by $H_2O_2$ and the most susceptible are the methionines at positions 411, 531, 602, 968, 1004, and 1144. Potency analysis by cell-based potency assay (CBPA) revealed a dose-dependent potency loss with increasing amounts of $H_2O_2$.

*Materials and Methods*

1. Materials and Reagents

Materials and reagents are shown in Table 8 below.

Table 8

| Item | Manufacturer | Catalog Number |
|---|---|---|
| 2 μm sterilizing filter, acrodisk | Pall | 4652 |
| Hydrogen peroxide | VWR | E882-10ML |
| DL methionine | Sigma | M2768-100G |

(continued)

| Item | Manufacturer | Catalog Number |
|---|---|---|
| Sodium phosphate monobasic, monohydrate | Macron Fine Chemicals | 7892-04 |
| Sodium phosphate dibasic, anhydrous | Macron Fine Chemicals | 7917-04 |

2. Dissolution of BoNT/A Drug Substance ("DS")

BoNT/A DS stored as a precipitate (starting concentration 2.5 mg/mL) was diluted with 40 mM sodium phosphate, pH 6.5 to a concentration of 0.5 mg/mL and incubated for at least 20 hours at 25 °C until sample was visibly clear. Dissolved samples were sterile filtered and aliquoted according to individual experiment requirements.

3. Oxidation of BoNT/A DS

Dissolved DS samples were treated with 0 ppm (control), 10 ppm, 50 ppm, or 100 ppm $H_2O_2$ for 20 hours at 25 °C. The concentration of BoNT/A DSwas kept the same in all samples by adding a constant volume of $H_2O_2$ (or water for control sample). Oxidation was quenched with the addition of a 10-fold molar excess of methionine (compared to the concentration of $H_2O_2$). Since oxidation of the protein (on residues like tryptophan) can affect the UV absorbance, the protein concentration of the control sample was determined by UV spectrophotometry and provided as a representative concentration of all samples. Samples were stored at $\leq$ -70 °C.

4. Sample Digestion and LC-MS/MS Analysis

Samples were denatured by heating at 99 °C in the presence of 0.05% RapiGestTM, reduced with dithiothreitol, alkylated with iodoacetamide, and digested with trypsin at an enzyme to protein ratio of 1:10 (weight trypsin/weight BoNT/A DS) for 18 hours at 37 °C. RapiGestTM was removed by acidification and subsequent centrifugation at 23,500 rcf. The resulting peptides were analyzed using an Agilent 1200 capillary HPLC workstation coupled to a Thermo LTQ-FT Ultra mass spectrometer. Twenty picomoles of each sample digest were injected for LC-MS/MS analysis. Mass spectrometry of the peptides eluting from the reversed phase column was performed using electrospray ionization in positive mode. The LC-MS/MS parameters used for the analysis of the digest samples are provided in Table 9. An accurate mass measurement of the precursor ions was obtained using the Fourier Transform Ion Cyclotron Resonance (FT) detector and the top four precursor ions from each MS scan were fragmented and analyzed in the linear ion trap in subsequent MS/MS scans. For the mass spectrometer, a full calibration of the instrument is performed quarterly, while the FT mass accuracy calibration is performed weekly using the calibration mixture recommended by the manufacturer. System performance was evaluated prior to the sample analysis by injecting two picomoles of a standard peptide mixture (Michrom, Auburn, CA).

Table 9 LC-MS Experimental Conditions

| Sample | Trypsin digests of BoNT/A DS |
|---|---|
| Sample load | 20 picomoles |
| Instrument | Agilent 1200 capillary HPLC (Cap pump S/N DE60555131) coupled with a Thermo LTQ-FT Ultra mass analyzer (S/N SN6126F) in the laboratory TL-M204. |
| Column | Agilent Zorbax 300 SB-C18, 1.0 x 150mm, 3.5 $\mu$m. 300 Å, at 45 °C |
| Mobile Phase A (MPA) | 0.1% formic acid in water |
| Mobile Phase B (MPB) | 0.1% formic acid in 80% acetonitrile |

(continued)

| Gradient | Time (min) | Flow rate ($\mu$l/min) | % MPA | % MPB |
|---|---|---|---|---|
| | 0 | 30 | 100 | 0 |
| | 10 | 30 | 100 | 0 |
| | 100 | 30 | 65 | 35 |
| | 140 | 30 | 40 | 60 |
| | 155 | 30 | 20 | 80 |
| | 160 | 30 | 10 | 90 |
| | 170 | 30 | 10 | 90 |
| | 175 | 30 | 100 | 0 |
| | 200 | 30 | 100 | 0 |

5. CBPA Analysis

The CBPA analysis was performed for the methionine-quenched $H_2O_2$-oxidized BoNT/A samples following the method described in Fernández-Salas et al. (2012) PLOS ONE 7(11):e49516. SiMa human neuroblastoma cells were cultured in collagen IV flasks in DSMZ's recommended medium. For optimal differentiation, SiMa cells were plated in 96-well plates at $5\times10^4$ cells/well in 100 $\mu$L differentiation medium (Minimum Essential Medium with 2 mM GlutaMAX™ I with Earle's salts, 0.1 mM Non-Essential Amino-Acids, 10 mM HEPES, 1x N2 supplement, and1x B27 supplement) for three days. SiMa cells were then treated with BoNT/A samples, followed by replacing with the medium with BoNT/A samples with fresh medium without toxin.

Cells were washed once with PBS and lysed in freshly prepared Triton X-100 Lysis Buffer (50 mM HEPES, 150 mM NaCl, 1.5 mM $MgCl_2$, 1 mM EGTA, 1% Triton X-100, and one tablet of EDTA-free protease inhibitors) on ice for 20 min. Lysed cells were centrifuged in the plate at 4000 rpm for 20 min at 4°C. For Western Blot analysis, the lysates were transferred to a 96-well PCR plate, 2x SDSPAGE loading buffer (Invitrogen) was added, and the plate was heated to 95°C for 5 minutes. The gels were run in 1x MOPSSDS running buffer (Invitrogen) at 200 V for 55 min. Proteins were transferred to nitrocellulose membranes (Bio-Rad) pre-wet in Western blot transfer buffer (Invitrogen) containing 20% Methanol (Burdick and Jackson). The Western blot transferred at 800 mA for 2 hours using the TE-62 transfer cell apparatus (GE Healthcare). Blots were blocked in 2% ECL blocking agent (GE Healthcare) in 1x TBS/0.1% Tween 20 (Bio-Rad) (TBS-T) for 1 hour at room temperature. The following primary antibodies were used: anti-SNAP25$_{197}$ polyclonal antibody diluted 1:1000, SMI-81 antibody (Sternberger Monoclonals Inc) diluted 1:5000 to evaluate the intact and cleaved SNAP25 products (i.e. SNAP25$_{206}$ and SNAP25$_{197}$ were detected). Antibodies were diluted in 2% blocking agent/TBS-T buffer and incubated overnight at 4°C with gentle shaking. Secondary antibodies were anti-rabbit or anti-mouse IgG H+L HRP conjugated (Invitrogen) diluted 1:5,000 or 1:10,000 in 2% blocking agent/TBS-T buffer for 1 hour at room temperature. The membranes were washed and exposed for 5 min to ECL Plus Western Blotting System Detection Reagents (GE Healthcare). Chemifluorescence was captured by scanning the blots in the Typhoon 9410 Imager (GE Healthcare) at $\lambda_{ex}$ 452/$\lambda_{em}$ 520. The intensity of the gel bands was calculated using ImageQuant TL software (GE Healthcare). The data was analyzed using SigmaPlot v 10.0 (Systat Software Inc.) or PLA2.0 (Stegmann Systems). Intensity values were plotted against concentration of the BoNT/A sample in log scale and fitted to a 4-parameter logistics function ($Y=Y_0+a/[1+(X/X_0)^b]$) without constraints. Based on the fitted curves the EC50 values, corresponding to "$X_0$", were determined.

For the ECL sandwich ELISA, MSD High Bind plates (Meso Scale Discovery) pre-spotted with anti-SNAP25$_{197}$ MAb 2E2A6 were blocked with 150 $\mu$L blocking buffer for 1 hour at RT. After blocking, the buffer was discarded and 25 mL of cell lysate were added to each well of the plate followed by incubation as detailed in text. Plates were washed with PBS-T, and SULFO-TAG NHS-Ester labeled detection pAb anti-SNAP25 antibody (Antibody to N-terminus of SNAP25, Cat# S9684, Sigma) in diluent buffer was added. Plates were sealed and shaken at room temperature for 1 h, washed with PBS-T, and 150 mL of 1x Read Buffer was added per well. Plates were immediately read on the SI6000 Image plate reader. For the chemiluminescence sandwich ELISA, white plates (Greiner) were coated with 100 $\mu$L/well of anti-SNAP25$_{197}$2E2A6 MAb at 4°C overnight. Plates were blocked with 2% ECL blocking with 10% goat serum for 1 hr at RT. Fifty microliters of cell lysate were added to each well and the plates were incubated at 4°C. S9684 anti-SNAP25 pAb conjugated with HRP was used for detection. The plates were developed with SuperSignal ELISA Pico 1:1 mixture (Pierce) and read at 395 nm on a Luminometer (Molecular Devices). Data was fitted to a 4-parameter logistics function ($Y=Y_0+a/[1+(X/X_0)^b]$).

Potential matrix effects of $H_2O_2$ and methionine on the CBPA assay have been considered. Since $H_2O_2$ either reacts to completion or is quenched by the excess methionine, there is no $H_2O_2$ remaining in the samples at the time of CBPA analysis. Any potential matrix effects of methionine are mitigated by the fact that the samples are diluted 30,000-fold with dilution medium prior to CBPA analysis. The highest concentration of methionine in a sample prior to dilution for CBPA analysis was 30 mM (methionine); therefore, the highest concentration of methionine added to treated cells after the 30,000-fold dilution with dilution medium was approximately 1 $\mu$M (methionine).

6. LC-MS/MS Data Analysis

The LTQ-FT Xcalibur (.raw) data files were submitted to Mascot Daemon version 2.1.5 (available on the World Wide Web at mascot/mascot) for database searching. The search parameters used for the database analysis are provided in Table 10.

Table 10 Mascot Database Search Parameters

| Parameter | Value |
|---|---|
| Database | NG_SProt |
| Enzyme | trypsin |
| Max. Missed Cleavages | 0 |
| Fixed Modifications | none |
| Variable Modifications | Carbamidomethy (C), Deamidated (N.Q). Dioxidation (C). Dioxidation (W), Oxidation (HW). Oxidation (M). Trioxidation (C)[1] |
| Monoisotopic Mass | yes |
| Peptide Tolerance | + 7 ppm |
| Peptide Charge | +1, +2, and +3 |
| MS/MS Ions Search | yes |
| MS/MS Tolerance | $\pm$ 1 Da |
| Data Format | Mascot generic |
| Instrument | ESI-FTICR |
| [1] C = cysteine, N = asparagine, Q = glutamine, W = tryptophan. M = methionine | |

For the quantification of relative percent oxidation at each site, the area of the extracted ion chromatogram peak of each oxidized and corresponding non-oxidized peptide was used with the following formula:

$$\% \text{ oxidation}_{(site\ n)}= \frac{(\text{peak area of the oxidized peptide})}{(\text{peak area of oxidized peptide} + \text{peak area unoxidized peptide})} \times 100$$

The percentages of increase in oxidation of each sample (at various amino acid residues) compared to the respective control and are calculated using the following formula:

$$\% \text{ increase in oxidation}_{(site\ n)}= \% \text{ oxidation}_{(site\ n)}(\text{treated sample}) - \% \text{ oxidation}_{(site\ n)}(\text{control sample})$$

*Results and Discussion*

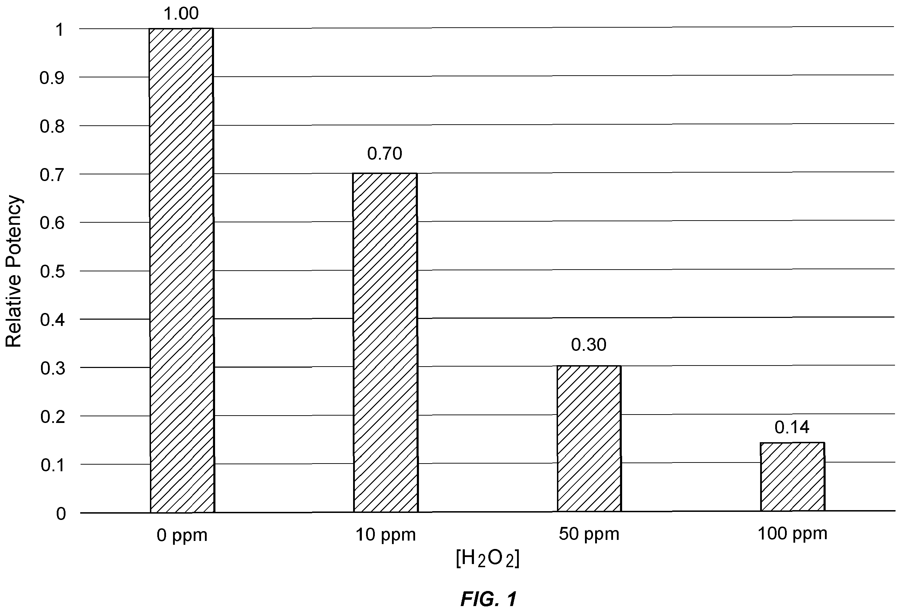

A study was performed to determine the potency effects and oxidation levels at various amino acids upon treatment with higher amounts of hydrogen peroxide. The results of the potency analysis of the BoNT/A DS samples oxidized with $H_2O_2$ at levels of 10 ppm, 50 ppm and 100 ppm (relative potency, compared to unoxidized control) are shown in FIG. 1.

The CBPA results of this study showed a loss in potency in response to treatment with 10 ppm $H_2O_2$ (approximately 30% loss). Further loss of potency was observed upon treatment with higher amounts of $H_2O_2$ with a total loss of approximately 70% and 85% potency after treatment with 50 ppm $H_2O_2$ and 100 ppm $H_2O_2$, respectively.

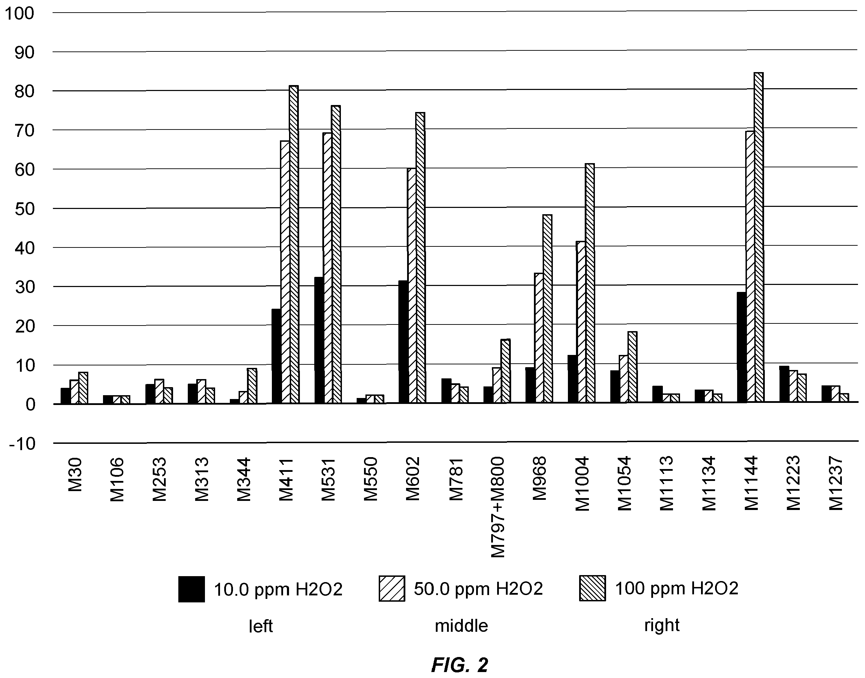

To determine the sites and amounts of $H_2O_2$-induced oxidation of the 150 kDa NT subunit of the BoNT/A complex, the negative control and oxidized samples were digested and the resulting peptides were analyzed by LC-MS/MS. The results of the LC-MS/MS analyses are shown in FIG. 2.

The mass spectrometry analysis identified six primary methionine oxidation sites on the 150 kDa NT (having a percent increase of oxidation > 20%): M411, M531, M602, M968, M1004, and M1144.

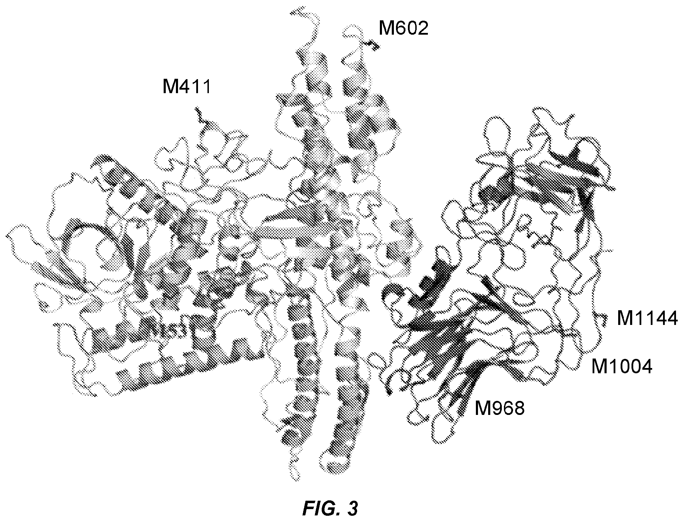

The results of these studies demonstrate that the potency of BoNT/A complex can be negatively impacted by oxidation by $H_2O_2$. The oxidation pathway of the 150 kDa NT subunit of BoNT/A by $H_2O_2$ has been determined and primarily includes six methionine residues at positions 411, 531, 602, 968, 1004, and 1144. FIG. 3 shows the location of the six methionine residues most prone to oxidation on the 150 kDa NT.

Residue M411 is part of the light chain and all other residues reside in the heavy chain: M531 on the loop region of the heavy chain, M602 on the membrane translocation domain, and M968, M1004, M1144 on the membrane binding domain.

The crystal structure of the BoNT/A receptor-binding domain in complex with the SV2C receptor has been solved (Benoit et al. (2017) Sci Rep. 7:43588). It has been shown that the interaction includes amino acids Thr1145, Thr1146 and Ser1294 on BoNT/A. Since M1144 is just prior to two of these amino acids, its oxidation can cause a decrease in receptor binding and thus, loss of potency.

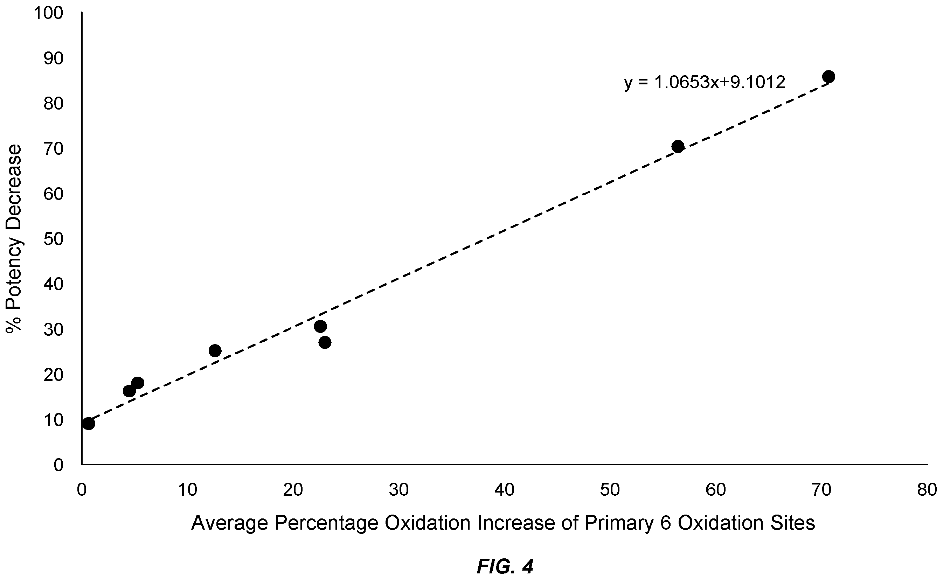

The extent of oxidation increase upon treatment with $H_2O_2$ was similar for the primary six oxidation sites (FIG. 2). The plot of the average percentage oxidation increase of the six primary oxidation sites versus percentage loss of potency shows a linear relationship (FIG. 4), with a slope of 1.1.

### 6.5. Example 5: Non-APF (Schantz) Process for Obtaining a *Botulinum* Toxin

This example sets forth the prior art Schantz process for obtaining *botulinum* neurotoxin. The process is a non-APF process using animal derived media and reagents (*i.e.,* beef blood agar plates for culturing, casein in the fermentation medium and use of Rnase and Dnase enzymes for botulinum neurotoxin purification). The Schantz process has about 16 to 20 major steps, for production scale work uses a 115 L fermentor and takes about 3 weeks to complete. The Schantz process is commenced by thawing a non-APF *Clostridium botulinum* master cell bank (MCB) vial to room temperature followed by four cultivation steps. First to select colonies with a suitable morphology, aliquots from the thawed MCB vial are streaked on pre-reduced Columbia blood agar (CBA) plates and anaerobically incubated for 30-48 hours at 34°C $\pm$ 1°C. Second, selected colonies are inoculated into 9 mL test tubes containing a casein growth medium for 6-12 hours at 34°C. The contents of the 9 mL tube with the most rapid growth and highest density (growth selection step) are then further cultivated through two step-up anaerobic incubations (the third and fourth cultivation steps), being a 12-30 hour incubation at 34°C in a 600 mL to 1 L seed cultivation bottle, followed by a cultivation in a 15 L to 25 L seed fermentor containing a casein growth medium for 6-16 hours at 35°C. These two step-up cultivations are carried out in a nutritive media containing 2% casein hydrolysate (a casein [milk protein] digest), 1% yeast extract and 1% glucose (dextrose) in water at pH 7.3.

The step-up cultivations are followed by a further incubation for 60-96 hours at 35°C in a commercial scale (*i.e.,* 115 L) production fermentor in a casein containing medium under a controlled anaerobic atmosphere. Growth of the bacterium is usually complete after 24 to 36 hours, and during the fermentation step carried out for about 65 to about 72 hours where most of the cells undergo lysis and release botulinum neurotoxin. It is believed that toxin is liberated by cell lysis and activated by proteases present in the media. A filtrate of the culture medium can be prepared using a single layer depth filter to remove gross impurities (*i.e.,* whole and ruptured cells) thereby obtaining a clear solution referred to as a clarified culture. Collection of botulinum neurotoxin from clarified culture is accomplished by lowering the pH of the clarified culture to pH 3.5 with 3M sulfuric acid to precipitate the raw toxin at 20°C (acidification precipitation). The raw botulinum neurotoxin is then concentrated (to achieve a volume reduction) by ultramicrofiltration (microfiltration) (referred to as MF or UF) followed by diafiltration (DF). A 0.1 $\mu$m filter is used for the microfiltration step.

The harvested crude or raw toxin is then transferred to a digestion vessel and stabilized by addition of the protease inhibitor benzamidine hydrochloride. Dnase and Rnase are added to digest (hydrolyze) nucleic acids. The toxin is then extracted with pH 6.0 phosphate buffer and cell debris removed by clarification. Hydrolyzed nucleic acids and low molecular weight impurities are then removed by further UF and DF steps. Next three sequential precipitation steps (cold ethanol, hydrochloric acid and ammonia sulfate precipitations) are carried out. The purified botulinum neurotoxin complex (bulk toxin) is stored as a suspension in a sodium phosphate/ammonium sulfate buffer at 2°C to 8°C.

Completion of this Example 5 Schantz (non-APF) process, including the harvesting and purification steps, takes about two to three weeks. The resulting bulk botulinum neurotoxin is a high quality suspension of 900 kDa botulinum toxin type A complex made from the Hall A strain of *Clostridium botulinum* with a specific potency of $\geq$2 X $10^7$ U/mg, an $A_{260}/A_{278}$ of less than 0.6 and a distinct pattern of banding on gel electrophoresis, and suitable for use for the compounding of a botulinum toxin pharmaceutical composition.

*Botulinum* neurotoxin can also be obtained from an APF, non-chromatographic process, as set forth in Example 7 of U.S. patent 7,452,697, the complete APF, non-chromatographic process (from beginning of culturing to end of all purification and processing steps) taking about two to three weeks to complete. Alternately, botulinum neurotoxin can also be obtained from an APF, chromatographic process, as set forth in Example 16 of U.S. patent 7,452,697, the APF, chromatographic process (from beginning of culturing to end of all purification and processing steps) taking a week or longer to complete.

### 6.6. Example 6: APF, Column Chromatographic Systems and Processes for Obtaining a *Botulinum* Neurotoxin

Rapid APF, anion-cation chromatographic based systems and processes are developed for obtaining high yield, high purity *botulinum* neurotoxin. The process of this Example 6 for production purposes (that is to obtain gram quantities of the final *botulinum* neurotoxin) uses a 20 L fermentation vessel and takes only 4-7 days, preferably about 4 to about 6 days, to complete all step of the process from initiation of culturing to completion of final purification and toxin storage. Apparatus utilized in the systems herein disclosed are discussed below. Chromatographic media processes are developed and are set forth herein. These chromatographic media processes use one, two, or three of the following: hydrophobic interaction chromatography (HIC), anion exchange chromatography, cation exchange chromatography. Specifically, the chromatographic media processes that are developed include: (1) a one media process that uses HIC; (2) a two media process that uses HIC followed by anion exchange chromatography; (3) a two media process that uses HIC followed by cation exchange chromatography; (4) a three media process that uses HIC followed by anion exchange chromatography followed by cation exchange chromatography; (5) a three media process that uses HIC followed by cation exchange chromatography followed by anion exchange chromatography; (6) a one media process that uses anion exchange chromatography; (7) a two media process that uses anion exchange chromatography followed by HIC; (8) a two media process that uses anion exchange chromatography followed by cation exchange chromatography; (9) a three media process that uses anion exchange chromatography followed by HIC followed by cation exchange chromatography; (10) a three media process that uses anion exchange chromatography followed by cation exchange chromatography followed by HIC; (11) a one media process that uses cation exchange chromatography; (12) a two media process that uses cation exchange chromatography followed by HIC; (13) a two media process that uses cation exchange chromatography followed by anion exchange chromatography; (14) a three media process that uses cation exchange chromatography followed by HIC followed by anion exchange chromatography; and (15) a three media process that uses cation exchange chromatography followed by anion exchange chromatography followed by HIC. The HIC removes impurities such as a 49 kDa impurity (which turns out to be a host cell glucose phosphate isomerase, as discussed below).

### 6.6.1. Preparation of Working Cell Bank

A new *Clostridium botulinum* cell bank is developed (for use to initiate the culturing step) without use of Columbia blood agar plates, and which removes the need for colony selection prior to cultivation and also eliminates the need to carry out the Shantz process step up tube cultivation and multiple seed (cultivation) steps.

For this purpose, a previously established Schantz master cell bank (MCB) is used to create an APF research cell bank (RCB) from which a new APF master cell bank (MCB) and a subsequent working cell bank (WCB) are generated. A research cell bank (RCB) is made from a colony from the Schantz (NAPF) MCB. To remove the animal-derived protein from the MCB vial, the cells are washed twice in APF medium containing 2% w/v SPTII (Soy Peptone type II), 1% w/v yeast extract, and 1% w/v glucose. The cells are plated on APF medium under strict anaerobic conditions using a Modular Atmosphere Controlled System (MACS) anaerobic chamber. An isolated colony is further expanded and stored in APF medium containing about 20% glycerol below -135°C.

The APF-MCB is made under GMP conditions by expanding the RCB into oxygen-free APF medium (200 mL, reduced for a minimum of 12 hours in an anaerobic chamber) and cultured in a MACS anaerobic chamber at 34.5°C $\pm$1°C (stirred at 60 rpm) until the $OD_{540}$ of the culture reaches 2.5+1.0 AU. Sterile glycerol is added to the resultant culture to a final concentration of about 20% after which the mixture is transferred into cryovials at 1 mL/vial (APF-MCB vials). The vials are flash frozen in liquid nitrogen, and then stored below -135°C. An APF-WCB is made under GMP conditions by expanding as above. The resultant APF cell banks are characterized for identity, purity, viability and genetic stability.

### 6.6.2. Upstream Steps (Culturing and Fermentation)

The Example 6 processes have two general stages: an upstream stage and a downstream stage. The upstream stage includes expansion of a starting cell line (growth and reproduction of *Clostridium botulinum* bacteria in a substantially APF culture medium), fermentation, harvest (removal of cellular debris) to provide a clarified, harvested culture that is then concentrated and diluted. Thus, in this example the steps of an exemplary three column process can include culturing, fermentation, harvest filtration, concentration, HIC, capture (anion) chromatography, polishing (cation) chromatography, buffer exchange, bioburden reduction and vial fill.

The upstream stage includes use of a culture medium in a 1 L bottle containing 400 mL of reduced (in an anaerobic chamber) seed APF culture medium (2% w/v SPTII, 1% w/v yeast extract, (adjusted to pH 7.3 with 1 N sodium hydroxide and/or 1 N hydrochloric acid prior to autoclaving)) 1% w/v sterile glucose added post autoclaving of culture media). The culture (seed) medium is inoculated with 400 $\mu$L of a thawed *Clostridium botulinum* WCB. Incubation/culturing occurs at 34.5°C $\pm$ 1.0°C with 150 rpm agitation in an anaerobic chamber.

When the optical density of the culture medium at 540 nm is 1.8 $\pm$ 1.0 AU, the entire contents of the 1 L bottle (approximately 400 mL) are transferred to a 20 L production fermentor containing APF fermentation medium adjusted with 1 N sodium hydroxide and/or 1 N hydrochloric acid post-steam sterilization to pH 7.3, fermentation medium composed of 3.25% w/v SPTII, 1.2% w/v yeast extract, 1.5% w/v sterile glucose (added post sterilization; sterilization, e.g. at about 122°C for 0.5 hour). The temperature and agitation are controlled at 35°C $\pm$ 1°C and 70 rpm, respectively. Nitrogen overlay is set at 12 slpm and headspace pressure set at 5 psig to maintain an anaerobic environment for cell growth. Fermentation pH and cell density are monitored by pH and online turbidity probes, respectively. The three phases for the production fermentation include exponential growth, stationary, and autolysis phases. Cellular autolysis, which releases active BoNT/A complex into the culture medium, is observed to occur consistently between 35 hours and the end of fermentation. At the end of fermentation, the culture is cooled to 25°C for harvest.

Once the fermentation medium is cooled to 25°C, the cell debris is separated from the botulinum neurotoxin type A complex containing lysate by depth filtration, first through a 5 - 0.9 $\mu$m nominal retention rating gradient pre-filter to remove cell debris, and then through a positively charged 0.8 - 0.2 $\mu$m nominal retention rating gradient to remove DNA (removal of up to about 80%). Both filters are rinsed together with 20 L of water for injection (WFI) before use. A minimum of 15 L of the filtrate is required for further processing, and any excess material is decontaminated after in-process sampling is complete. The filtrate is stored at 4°C if not immediately processed by ultrafiltration.

Within a biosafety cabinet (BSC) the filtrate from the harvest step is concentrated from 15 L to 5 $\pm$ 0.5 L using a hollow fiber, tangential flow filtration (TFF) membrane from GE Healthcare. The ultrafiltered material is then diluted with 10 mM sodium phosphate pH 6.5 buffer to a final volume of 20 L. This material is purified by use of one chromatography column, two chromatography columns or three chromatography columns (*see* the Downstream Steps section). The diluted, ultrafiltered harvest material is stored at 4°C if not immediately processed by purification.

In the Schantz process the culture step is ended and the fermentation step begins based on time and visual observation of culture growth. In contrast, in the Example 6 processes determination of when to end the culturing step is based on analysis of culture fluid optical density, which ensures that the culture is in the logarithmic growth phase at the time of commencement of the fermentation step, and permits reduction of duration of the culturing step to about 8 hours to about 14 hours. The OD parameter terminated culture step maximizes the health of the cultured cells and encourages robust and abundant *botulinum* toxin resulting from the fermentation step. The average optical density (at 540 nm) of the culture medium at conclusion of culturing is 1.8 AU. The average duration of the fermentation step is 72 hours and the average final turbidity ($A_{890}$) of the fermentation medium at conclusion of the fermentation step is 0.15 AU. The average amount of *botulinum* toxin type A complex present (as determined by ELISA) in the 20 L fermentation medium (whole broth) at the end of the fermentation step for is about 64 $\mu$g botulinum toxin type A complex/mL fermentation medium.

The harvest step uses depth filtration to remove cell debris and nucleic acids, followed by ultrafiltration and dilution to prepare the fermentation medium for the next step in the process. This harvesting/cell debris clearing is fundamentally different from the Schantz harvest process, which uses precipitation by acidification followed by microfiltration and diafiltration to concentrate and exchange buffers in preparation for further processing.

### 6.6.3. Downstream Steps (Purification)

Downstream steps include purification by one chromatography column, two chromatography columns, or three chromatography columns (for example, capture of the *botulinum* neurotoxin on an anion exchange column, elution from the column and further separation from impurities by polishing on a cation exchange column, and preferably (in the three column process), passage of eluent containing desired botulinum neurotoxin through a third column, preferably a hydrophobic interaction column (*e.g.* chromatography), before (preferably) or after the two-column process), followed by concentration and buffer exchange using tangential flow filtration (TFF), and bioburden reduction (*e.g.* by further filtration using a 0.2 $\mu$m filter) to a final *botulinum* neurotoxin type A complex optimized for cold storage, preferably freezing, and eventual compounding into a *botulinum* neurotoxin type A complex pharmaceutical composition. The sequence of the chromatography and filtration stages is intended to remove product and process-related impurities, to remove potential adventitious agents and to control the *botulinum* neurotoxin type A complex concentration and buffer matrix of the final botulinum neurotoxin type A in order to provide a more stable drug substance.

Several embodiments of exemplary two-column and three-column downstream processes are described below. Additional embodiments are contemplated in which the order of the columns is changed, and the procedures are adjusted accordingly as deemed appropriate in the opinion and judgment of a person skilled in the art, but which are otherwise similar to or essentially identical to the embodiments described below. Further embodiments of one-column downstream processes are also contemplated, which embodiments involve the use of only one chromatography column but are otherwise similar to or essentially identical to the embodiments described below. Specifically, this example contemplates a three-column downstream process that involves (1) the use of HIC followed by anion exchange chromatography followed by cation exchange chromatography, (2) the use of HIC followed by cation exchange chromatography followed by anion exchange chromatography, (3) the use of anion exchange chromatography followed by HIC followed by cation exchange chromatography, (4) the use of anion exchange chromatography followed by cation exchange chromatography followed by HIC, (5) the use of cation exchange chromatography followed by HIC followed by anion exchange chromatography, or (6) the use of cation exchange chromatography followed by anion exchange chromatography followed by HIC. This example also contemplates a two-column downstream process that involves (1) the use of HIC followed by anion exchange chromatography, (2) the use of HIC followed by cation exchange chromatography, (3) the use of anion exchange chromatography followed by HIC, (4) the use of anion exchange chromatography followed by cation exchange chromatography, (5) the use of cation exchange chromatography followed by HIC, or (6) the use of cation exchange chromatography followed by anion exchange chromatography. This example also contemplates a one-column downstream process that involves the use of HIC, the use of anion exchange chromatography, or the use of cation exchange chromatography.

A first detailed embodiment of an exemplary three-column downstream process carried out is as follows. Clarified (diluted) ultrafiltered material (20 L, as disclosed above) is passed through a POROS® 50HQ anion exchange chromatography resin, the captured botulinum neurotoxin is eluted from the anion exchange column and then run through a POROS® 20HS cation exchange chromatography resin, the eluent from which is run through a Phenyl Sepharose HP chromatography resin. Eluent from the HIC column is subjected to 100 kDa tangential flow filtration, followed by 0.2 μm filtration. The resulting botulinum neurotoxin type A complex is frozen for storage.

In this first embodiment of the exemplary three-column downstream process, the first chromatography step of the downstream process uses a POROS® 50HQ anion exchange chromatography resin packed into a column with an inner diameter of about 8 cm and a column height of about 15 cm. The entire POROS® 50HQ column operation is completed at ambient temperature, and the flow is in the downward direction. The *botulinum* neurotoxin type A complex is eluted from the anion column using a pH step change where the more negatively charged components such as nucleic acids (*e.g.* DNAs and RNAs) and other host cell proteins remain bound to the anion exchange column.

Particulars of the anion exchange step are: use of the POROS® 50HQ column using 0.1 N sodium hydroxide for a minimum contact time of 30 minutes (at least about 3 column volumes, at 230 cm/hour). The column is then equilibrated with a 50 mM sodium phosphate, pH 6.5 buffer (at least 5 column volumes). Next the clarified ultrafiltered and diluted material (i.e. processed lysate APF fermentation material) is loaded at 230 cm/hour onto the POROS® 50HQ anion exchange column, followed by washing with at least about 20 column volumes of 50 mM sodium phosphate, pH 6.5 at 230 cm/hour until absorbance at 280 nm of column effluent decreases to 0.10 AU, followed by eluting with 50 mM sodium acetate, pH 4.8 at 230 cm/hour. The product pool is collected, when the absorbance at 280 nm ($A_{280}$) increases to at least about 0.15 AU and through the peak maximum to equal or less than about 0.2 AU on the trailing edge, into a vessel containing 1 column volume of 50 mM sodium acetate, pH 4.8. This elution pool is stored at about 2°C to about 8°C for up to 48 hours.

The second chromatography step in this first embodiment of the exemplary three-column downstream process of this Example 6 uses a POROS® 20HS cation exchange chromatography resin packed into a column with an inner diameter of 8 cm and a column height of 5 cm. The entire POROS® 20HS column operation is completed at ambient temperature, and the flow is in the downward direction. The *botulinum* neurotoxin type A complex associates with the POROS® 20HS column resin. The *botulinum* neurotoxin type A complex is then eluted from the column using a salt step change. The product-related impurities are eluted with the wash buffer and decontamination solution.

Particulars of the cation exchange step are: use of the POROS® 20HS column using 0.1 N sodium hydroxide solution for a minimum contact time of 30 minutes (at least about 3 column volumes, at 230 cm/hour). The column is then equilibrated with a 50 mM sodium acetate, pH 4.8 buffer (at least about 5 column volumes). Next the POROS® 50HQ product pool (collected as described above, fresh or from refrigeration) is loaded onto the POROS® 20HS column. The column is then washed with a 50 mM sodium acetate, pH 4.8 buffer (at least about 3 column volumes) and then washed again with a 50 mM sodium acetate, 150 mM sodium chloride, pH 4.8 buffer. The *botulinum* neurotoxin type A complex is eluted from the POROS® 20HS column with a 50 mM sodium acetate, 250 mM sodium chloride, pH 4.8 buffer at 200 mL/min, the eluate is diverted into a bioprocess collection bag (containing 1 column volume of 50 mM $NaH_3C_2O_2$, pH 4.8) when the $A_{280}$ increases to about ≥ 0.1 AU through peak maximum until the $A_{280}$ of the trailing edge of the elution peak decreases to a trailing edge value of ≤ 0.1 AU. The POROS® 20HS product pool is stored in the collection bag at ambient temperature for up to about 6 hours.

In this first embodiment of the exemplary three-column chromatography media process of this Example 6, eluent from the second (cation exchange) column is passed through a HIC column. The HIC column used is a Phenyl Sepharose HP hydrophobic interaction chromatography resin packed into a column with an inner diameter of about 8 cm and a column height of about 5 cm. The entire Phenyl Sepharose HP column operation is completed at ambient temperature, and the flow is in the downward direction. The *botulinum* neurotoxin type A complex is eluted from the column using a decreasing salt step change. The impurities are eluted during the load and with the wash buffer and decontamination solution.

Particulars of the hydrophobic interaction chromatography step are:
a Phenyl Sepharose HP column is initially sanitized with a 0.1 N sodium hydroxide solution for a minimum contact time of 30 minutes (with at least about 3 column volumes of a 0.1 N sodium hydroxide solution at 200 cm/hour). The column is then equilibrated with at least about 5 column volumes of 50 mM sodium acetate, 0.4 M ammonium sulfate, pH 4.8 buffer. Next the POROS® 20HS (cation exchange column) product pool (from above) is combined 1:1 with a 50 mM sodium acetate, 0.8 M ammonium sulfate, pH 4.8 buffer and loaded onto the Phenyl Sepharose HP column. The column is first washed with at least about 3 column volumes of a 50 mM sodium acetate, 0.4 M ammonium sulfate, pH 4.8 buffer, and then washed with a 50 mM sodium phosphate, 0.4 M ammonium sulfate, pH 6.5 buffer. *Botulinum* neurotoxin type A complex is eluted from the column with a 10 mM sodium phosphate, 0.14 M ammonium sulfate, pH 6.5 buffer. The eluate is diverted into a bioprocess collection bag when the $A_{280}$ increases to $\geq 0.05$ AU. The eluate is collected until the $A_{280}$ of the trailing edge of the elution peak decreases to a value of $\leq 0.05$ AU. The Phenyl Sepharose HP product pool is stored in the collection bag at ambient temperature for up to 6 hours.

A tangential flow filtration system is used to concentrate and diafilter the Phenyl Sepharose HP chromatography step product pool into the drug substance formulation buffer. Pall® Filtron Minimate cassettes with a 100 kDa molecular weight cut off membrane are used for the concentration and diafiltration steps. The formulated material is then passed through a Pall Mini Kleenpak ® 0.2 μm filter to reduce the potential bioburden. The UF/DF step concentrates the Phenyl Sepharose HP product pool (eluent of the HIC column) to a BoNT/A complex concentration of 0.7 g/L and diafilters the concentrated material with a 10 mM potassium citrate, pH 6.5 buffer.

Particulars of the ultrafiltration/diafiltration process used are as follows. The UF/DF unit and Pall 100 kDa polyether sulfone membrane is initially flushed with a minimum of 5 L of water for injection (WFI) to remove the packing solution and sanitized with a minimum of 200 mL of a 1 N sodium hydroxide solution under recirculation conditions for a minimum of 10 minutes, preferably at least 30 minutes, to sanitize the UF/DF unit. Next the membrane and UF/DF system are equilibrated with sufficient volumes of the 10 mM potassium citrate, pH 6.5 formulation buffer until permeate and retentate pH is pH 6.5. After that the Phenyl Sepharose HP product pool is loaded onto the Minimate® tangential flow filtration cassette and the HIC eluate concentrated to 0.7 g/L. Following the concentration step, the retentate pool is diafiltered against a minimum of 5 diafiltration volumes of the drug substance formulation buffer (10 mM potassium citrate, pH 6.5) at a transmembrane pressure of 7.5 psig (pounds per square inch gauge). The permeate outlet is then closed and the UF/DF system run for at least 2 minutes and the system rinsed with 50 mL of 10 mM potassium citrate, pH 6.5 formulation buffer. After the rinse, the concentration of BoNT/A complex in the retentate pool is determined by measuring the offline $A_{278}$ and based on the $A_{278}$ reading, the concentration of the retentate pool is adjusted to 0.5 g/L with 10 mM potassium citrate, pH 6.5 buffer. The concentration-adjusted retentate pool is then filtered through a Pall Mini Kleenpak™ 0.2 μm filter to reduce potential bioburden. The filtered concentration-adjusted retentate pool is stored in a collection bag at 2°C - 8°C for up to 2 days.

The final purified *botulinum* neurotoxin type A complex obtained is filled into 1 mL Nunc® cryovials at 700 μL per vial and stored frozen. The filling operation is carried out in a class 100 biosafety cabinet at ambient temperature.

The downstream process (including use of 1 or 2 or 3 chromatography columns) is completed in only 1 to 3 days and the *botulinum* neurotoxin type A complex obtained is stored frozen in a potassium citrate, pH 6.5 buffer at a concentration of 0.5 g/L as a solution. In comparison, the Schantz downstream (toxin purification) process uses multiple filtration, precipitation, extraction and centrifugation steps to purify the *botulinum* neurotoxin type A complex and requires 1-2 weeks to complete just the downstream steps, and the resultant drug substance (recovered *botulinum* neurotoxin) is stored refrigerated as an ammonium sulfate suspension at a concentration of approximately 2.7 g/L. The use of chromatography instead of precipitation and the reduced processing time results in a significantly improved, consistent downstream process, as herein disclosed.

In accordance with one aspect, concentrations of vegetable-based products, such as soy-based products, can be Soy Peptone Type II Hy-Soy® or SE50MK(a Kosher soy peptone) in culture and fermentation media. Hy-Soy® in the seed culture medium can range between 10-200 g/L. Preferably, the concentration of Hy-Soy® in the seed medium ranges between 15-150 g/L. Most preferably, the concentration of Hy-Soy® in the seed medium is approximately between about 20-30 g/L or an amount therebetween. The concentration of glucose in seed medium can range between 0.1 g/L and 20 g/L. Preferably, the concentration of glucose ranges between 0.5-15 g/L. Most preferably, the concentration of glucose in the culture medium is approximately 10 g/L. Yeast extract amounts can be from about 5-20 g/L, more preferably from about 10-15 g/L or an amount therebetween. For example, the pH of the culture medium prior to growth of *Clostridium botulinum* can be approximately pH 7.0-7.5, or therebetween, preferably pH 7.3.

As an example, Hy-Soy® amounts in the production fermentation medium can range between 10-200 g/L. Preferably, the concentration of Hy-Soy® in the fermentation medium ranges between 15-150 g/L. Most preferably, the concentration of Hy-Soy® in the fermentation medium is approximately between about 20-40 g/L or an amount therebetween. The concentration of glucose in fermentation medium can range between 0.1 g/L and 20 g/L. Preferably, the concentration of glucose ranges between 0.5-15 g/L or an amount therebetween. Not necessarily, but as above, the glucose can be sterilized by autoclaving together with the other components of the fermentation medium. The pH level of the fermentation medium prior to growth can be pH 7.0-7.8, preferably about 7.0-7.5 or therebetween, more preferably pH 7.3.

An embodiment of an exemplary two-column downstream process comprises the following steps: (a) culturing bacteria, such as *Clostridium botulinum* bacteria from an APF WCB vial, in a seed/culturing bottle, (b) then fermenting *Clostridium botulinum* bacteria in a fermentor (toxin production fermentor) having APF fermentation medium to expand the cell line, proceeding with fermentation and botulinum toxin production until a desired cell lysis phase is reached. Next, (c) harvesting (*e.g.* clarifying by filtration,) the APF fermentation medium to obtain a harvested fermentation medium, (d) proceeding with concentration and dilution resulting in a diluted harvested fermentation medium that is (e) passed through a capture column to remove impurities, (f) contacting eluent from the capture column with a polishing column to further remove impurities, and optionally a second polishing column (g) concentration and buffer exchange of the polishing column eluent, (h) followed by bioburden reduction filtration and the (i) filling of vials.

In one example, the fermentation volume is 20 L, the total process time for all steps is only 4 to 6 days, and high botulinum neurotoxin yield is obtained.

The following provides more details of a particular embodiment within the scope of our invention. The fermentation step is carried out in APF medium using a 30 L stainless steel fermentor.

In this example below, a much-reduced volume of fermentation medium is used while still providing a high yield of high potency *botulinum* neurotoxin type A complex. By using the following protocol, only 20 L or less, for example, of APF fermentation medium is required, instead of the typically larger, previous volumes (*e.g.* 115 L) of fermentation medium required for producing commercially useful amounts for obtaining a *botulinum* neurotoxin.

The MACS anaerobic workstation (Don Whitley) with airlock provides an oxygen-deficient environment in which to manipulate anaerobic organisms. Access to and egress from the chamber is via a porthole system, comprises of inner and outer doors. The unit is temperature controlled to maintain a user setting within the chamber. A humidistat-controlled condensing plate ensures the effective removal of excess moisture in the chamber. The chamber is illuminated for operator use and alarm for: low gas pressure, continuous gas flow, and loss of power conditions. The chamber is equipped with a HEPA filter to reduce viable and non viable particulate levels in the anaerobic chamber. Anaerobic conditions are maintained utilizing the "Anotox" and Palladium Deoxo "D" Catalyst atmospheric scrubbing system. Condensate water from the condensing plate is collected and piped to an external reservoir where it is removed.

As disclosed above, an APF process is used for preparation of an APF WCB, having cell bank vials stored below -135°C. An APF WCB cell bank vial is thawed at room temperature for about 15 min before culture medium inoculation, followed by a single cultivation step as disclosed above to establish a "seed" culture. This is carried out in a modular atmospheric controlled system utilizing aseptic techniques throughout, to minimize bioburden. The modular atmospheric controlled system is cleaned before undertaking inoculation of the completed seed culture vial with APF WCB vial contents. Culture medium is prepared using 1 N hydrochloric acid and 1 N sodium hydroxide (for pH adjustment), D(+) Glucose, Anhydrous (Mallinckrodt Baker, Cat# 7730, 4.00 g), Soy Peptone Type II (SPTII) (Marcor, Cat #1130, 8.00 g), Water for Injection (WFI) 400.0 mL and Yeast Extract (YE) (BD Cat #212730, 4.00 g). The soy peptone Type II and yeast extract solution is made by measuring 300 mL of WFI with a 500 mL graduated cylinder and poured into a seed culture bottle. The seed culture bottle is placed onto a stirrer and the stirrer activated. 8.00 g of SPTII and 4.00 g of yeast extract is added to the seed culture bottle and mixed until dissolved. If dissolution is not complete after mixing, the mixture will be heated on low setting. The pH is measured and adjusted to about 7.30 $\pm$ 0.05. The medium solution is brought up to about 360 mL with WFI. The seed culture bottle is adequately vented to allow steam and gas transfer. A 10% Glucose solution (w/v) is prepared by measuring about 30 mL of WFI with a 100 mL graduated cylinder and placed into the pre-assembled glucose addition bottle, which is placed onto a stirrer and the stirrer activated. About 4.00 g of glucose is added to the glucose addition bottle and mixed until dissolved (low heat is used if necessary to a dissolution) and qs (quantity sufficient) glucose solution to 40 mL with WFI. The glucose addition is then capped loosely with vent cap. Both the glucose and seed culture bottles are autoclaved at 123°C for 30 minutes for sterilization. After sterilization, both items are removed from the autoclave and left to cool in a bio-safety cabinet. After cooling aseptically, 10% of the glucose solution is transferred into the seed culture bottle containing the yeast extract and soy peptone II solution and mixed, thereby providing a completed seed culture bottle.

This completed seed culture bottle is placed into the pre-cleaned MACS (wherein a prepared anaerobic indicator is placed). The cap of the completed seed culture bottle is loosened. The completed seed culture bottle is then placed on a stir plate within the MACS (stir plate activated to about 150 rpm) and the medium in the completed seed culture bottle is reduced for a minimum of 12 hours at about 34.5°C +/- 1°C within the MACS, after which a 1 mL medium blank is sampled for optical density measurement (for biomass determination at 540 nm). Afterwards, the completed seed culture bottle, in the MACS (anaerobic) is inoculated. An APF WCB culture vial is obtained from the frozen cell bank and brought into the MACS. The vial is thawed for about 10-15 minutes, after which about 400 µL of the vial contents are placed directly into the medium in the completed seed culture bottle. The cap on the completed seed culture bottle is loosened completely and the cap is rested on top of the bottle and the stir pace is set to 150 rpm. After at least about 11 hours of incubation in the MACS, fermentation production is undertaken, as described below.

Probes (*e.g.* redox probe, pH probe, turbidity probe, *e.g.* by Broadley James and Optek) and sequence configuration of the fermentor, such as a 30 L stainless steel fermentor, are checked and calibrated, and inserted into their respective fermentor ports and tightened in place. For example, a fermentor can be a ABEC 30 L (VT) Fermentor System consisting of a 30 L volume fermentor vessel, an agitator drive system, piping assembly for utility connections (CIP, clean steam, CDA, Nitrogen, Oxygen, Process Chilled Water, bio-waste, and plant steam), instrumentation (pH, temperature, pressure, ReDox, optical density, and mass flow), and four peristaltic pumps. The bottom mounted agitator speed is controlled using an Allen-Bradley variable frequency drive (VFD). Semi-automatic and automatic control of the system is handled by an Allen-Bradley ControlLogix PLC with programming. The system is designed to provide closed-loop PID (proportional-integral-derivative) control of culture temperature, pressure, pH, and redox during fermentation operations. An Allen-Bradley DeviceNet® (an open device level network) is utilized for control and communication with devices and sensors on the skid.

For sterile hold, equilibrium, run and harvest modes, agitation, temperature, pressure and Nitrogen overlay are operated with the following set points.

For sterile hold and equilibrium mode:

| Controlled Parameter | Set Points and Range |
|---|---|
| Agitation | 100 rpm ± 10 |
| Nitrogen Overlay | 12 SLPM ± 2 |
| Fermentor Pressure | 5 psig ± 1 |
| Fermentor Temperature | 35 ± 1°C |
| Redox | -390 to - 150 mV |

For RUN mode:

| Controlled Parameter | Set Points and Range |
|---|---|
| Agitation | 70 rpm ± 5 |
| Nitrogen Overlay | 12 SLPM ± 2 |
| Fermentor Pressure | 5 psig ± 1 |
| Fermentor Temperature | 35 ± 1°C |

For Harvest mode:

| Controlled Parameter | Set Points and Range |
|---|---|
| Agitation | 150 rpm ± 10 |
| Nitrogen Overlay | 10 SLPM ± 2 |
| Initial Fermentor Pressure | 0 psig |
| Fermentor Temperature | 25 ± 1°C |

To prepare fermentation medium, material needed include D(+) Glucose, Anhydrous (Mallinckrodt Baker, Cat# 7730, 300.0 g), Soy Peptone Type II (SPTII) (Marcor, Cat #1130, 650.0 g), Water for Injection (WFI, 13 L) and Yeast Extract (YE) (BD Cat #212730, 240.0 g), along with standard balances, a carboy (20 L, for example), glass bottle (5 L), graduated cylinders, stir bars and stirrers. About 10 L of WFI is added into the carboy along with a stir bar. The carboy is placed onto a stirrer and the stirrer is activated, after which about 650.0 g of soy peptone type II is added, along with about 240.00 g of YE. The fermentation medium is q.s. (quantity sufficient) to 13 L with WFI, and the carboy is capped. A 10% glucose solution (w/v) is then prepared by adding about 2 L if WFI into a glass 5 L bottle (with stir bar therein). Placed onto a stirrer and with the bar spinning, about 300.00 g of glucose is added into the bottle, and mixed until dissolved. The glucose solution is q.s. to 3 L with WFI and the bottled capped, thus providing a 10% glucose solution.

The fermentation medium in the carboy is added to the fermentor and pre-steam in place fermentor volume recorded and the fermentation sequence of operation is advanced. At the end of the SIP (steam in place)(122°C, +/- 1°C), the post-SIP fermentor volume is noted. A glucose addition assembly, comprising a vessel having tube therefrom with and in-line 0.2 $\mu$m filter (PALL Corp.) and peristaltic pump, is connected to the fermentor and the line is subjected to SIP and allowed to cool. An addition valve port is opened and about 3 L of glucose (filter sterilized) is added, and the appropriate amount of WFI (filter sterilized) to q.s. the total fermentor volume to 20 L is added to the glucose addition bottle and pumped into the fermentor through the same glucose filter line. The addition valve port is closed. The production fermentation medium has its pH adjusted thereafter, to about pH 7.3 +/- 0.05, with sterile 1 N sodium hydroxide or 1 N hydrochloric acid, utilizing SIP of addition lines, as required. Afterwards, parameters for sterile hold are set and held for about 12 hours before inoculation. The medium's starting glucose concentration is measured using a metabolite analyzer and glucose concentration recorded.

As stated above, at the end of seed culture incubation (about 11 $\pm$ 1 hours), 1 mL of sample is taken for optical density (OD) measurement. OD is measured offline at 540nm using a spectrophotometer and if within the appropriate range the OD value is recorded and culture is used for fermentation. The fermentor turbidity probe is accordingly zeroed. The seed inoculum bottle, from the anaerobic chamber, is brought over to the fermentor and a seed inoculum transfer assembly (a seed vessel with APF culture medium therein, the vessel having a culture inoculum transfer line with a sterile Kleenpak™ Connector assembly available from PALL Corp. or Millipore). The seed inoculum transfer line is then fixed to a peristaltic pump and the inoculum transfer line with sterile Kleenpak connector is connected to the fermentor. The fermentor pressure is lowered to 2 psig and entire volume of the seed inoculum bottle is pumped into the fermentor. At the end of inoculation, the online Absorbance Units (AU) from the fermentor is recorded, fermentor parameters are set to RUN mode and time is recorded.

Fermentation then proceeds (fermentation runs can be from about 60 hours to about 80 hours, preferably from about 68 hours to about 76 hours, most preferably for about 72 hours) while samples are taken from the fermentor, at 24 and 48 hours, for example, while maintaining aseptic conditions. Tests that are run on at least one sample taken during fermentation can include, but are not limited to, off-line optical density measurements, glucose measurements, ELISA, SDS-PAGE, Western blot, for example. At the end of the fermentation (end of fermentation broth volume is from about 18-19 L, for example), a sample may be taken (for testing by, for example, off-line optical density measurements, glucose measurements, ELISA, SDS-PAGE, western blot and DNA/RNA quantification.

At the end of the fermentation, online optical density, EFT (elapsed fermentation time), and fermentation end time is recorded, as well as agitation rpm, temperature in °C, pressure psig and Nitrogen overlay slpm and redox mV. Next, the production fermentation broth is subjected to harvesting, *i.e.* the production fermentation broth is clarified through filtration whereby, for example, about 15 L of filtrate is collected. The fermentation parameters are set for HARVEST and the filter assembly for clarification is prepared (CUNO, 3M filtration) which includes a pre-filter, depth filter and at least one pressure gauge. The pre-filter and depth filter are flushed with about 20 L of water for injection. After flushing, the filtration assembly is attached to the harvest/drain port of the fermentor. The fermentor temperature is decreased to about 25°C, after which clarification of the fermentation broth begins (record clarification start time, initial online OD, initial pH, initial temperature and initial volume of fermentor). The pressure in the fermentor is increased at a rate of about 1 psi (pound per square inch) about every 10 minutes during filtration, until a pressure of about 6 psi is reached, at which the pressure is held until the end of harvesting. This filter removes approximately 80% of the RNA/DNA in the APF fermentation medium (the remainder essentially removed during later chromatography steps, as discussed below), thus doing away with prior reliance/use of RNase and/or DNase to remove such components from the fermentation broth. Process parameters, such as pre-filter inlet pressure, depth filter inlet pressure, fermentor pressure, agitation and filtrate volume are monitored at every 2 L of filtrate collected, at the end of which the clarification end time and volume of filtrate collected is recorded. Following completion of harvest step, the systems are decontaminated and cleaned.

The filtrate carboy is brought into the BSC for sampling, from which about ≤10 mL of filtrate is sampled for offline OD measurements and other analysis (*e.g.* ELISA, SDS-PAGE, DNA/RNA and western blot).

The filtrate is then subjected to ultrafiltration/dilution. A tangential flow filter (TFF) unit assembly is assembled. The TFF unit is rinsed for about 90 minutes with WFI at a preferred rate of about 2 L per minute and then the TFF unit is sanitized by running 0.1 N sodium hydroxide (re-circulated) therethrough for about 60 minutes, after which 1 L of 10 mM sodium phosphate buffer, pH 6.5 is run therethrough, followed by a rinse with WFI for about 30 minutes. The filtrate from the harvest step (about 15 L) is then passed through the TFF (this is carried out in a bio-safety cabinet), concentrating the filtrate down to about 5 L +/- 0.5 L (the concentration step proceeds at about 2 L per minute and at a trans-membrane pressure of about 5 psig). A sample of the permeate can be taken and subjected to ELISA, dsDNA, SDS-PAGE and western blot tests, for example. Once concentrated to about 5 L +/- 0.5 L, the retentate pool is then diluted up to about 20 L with about 15 L of sterile filtered 10 mM sodium phosphate buffer, pH 6.5, through the TFF, at about a rate of 2 L per minute. A sample can be then again be taken and subjected to ELISA, DNA/RNA, SDS-PAGE and western blot tests, for example. The ultrafiltration/dilution material (retentate) is stored at 4°C.

Following use all systems are decontaminated using either 1N sodium hydroxide or sterilization (steam) temperatures and cleaned.

The following materials, equipment and procedures are used to make the solutions, buffers, etc, set forth below for use in an exemplary process, that is in the purification of the fermentation medium obtained from the Example 6 processes so as to obtain a purified *botulinum* neurotoxin type A complex. Exemplary buffers utilized (filtered through a 0.2-micron vacuum filter and their conductivity measured in mS/cm, for recordkeeping) include: 10 mM sodium phosphate, pH 6.5; 50 mM sodium phosphate, pH 6.5; 50 mM sodium acetate, pH 4.8; 50 mM sodium acetate, 170 mM sodium chloride, pH 4.8; 50 mM sodium acetate, 250 mM sodium chloride, pH 4.8; 50 mM sodium acetate, 1 M sodium chloride, pH 4.8; 50 mM sodium acetate, pH 4.0 and 10 mM citrate, pH 6.5.

The following is an example of operations for purification and obtaining *botulinum* neurotoxin type A from the Example 6 processes. All product-contact parts are designed and constructed to ensure that they are non-reactive and non-absorptive. Additionally, all equipments are designed to allow the utilization of single use disposable systems or are designed and constructed to facilitate sanitization, cleaning and decontamination as per documented, validated methods. The systems or skids are designed to be non-product contacting while the flow paths are designed to be single use disposable, including the chromatography columns and the all associated tubing. Chromatography components are obtained from AlphaBio and UF/DF components are obtained from Scilog Inc. The chromatography set ups used include a peristaltic pump for solution delivery with variable speed drive, inlet valve manifold with 5 inlets, a column valve manifold with an array of 3 automated valves, outlet valve manifold with 3 outlets, column effluent monitoring, including pH, conductivity, and UV, peak collection based on UV absorbance, and instrumentation and controls required to complete the purification operations. The control system has both the software and hardware designed to control the purification process. Commands and data are entered via a HMI (Human Machine Interface) terminal. The operator initiates all automated process functions by commands at the HMI and monitors and adjusts process parameters such as feed flow rates, pressure, conductivity, pH, UV absorbance and individual valve positions.

The UF/DF system includes a recirculation pump, diafiltration pump, 2 balances and a tangential flow filter (TFF) holder. The recirculation pump interfaces with 3 disposable pressure sensors and one of the balances (located under the permeate reservoir) to control the flow rate to maintain a defined transmembrane pressure and stop, based on the weight of the permeate reservoir. The diafiltration pump interfaces with the second balance (located under the retentate reservoir) to start and stop, based on maintaining a constant weight of the retentate reservoir.

After concentration and dilution of retentate material from the harvesting step (harvesting the animal protein free fermentation medium), the material is loaded onto an anion exchange column. The following is the procedure used for packing and testing the anion exchange column useful in the exemplary Example 6 two-column process.

Pre-packed columns are used for all three chromatographic steps. First, feed material (harvested APF media that has been subjected to ultrafiltration/dilution) is passed through the anion exchange column (Poros 50HQ, from ABI as described above). At least 5 column volumes (CVs) of 50mM sodium phosphate, pH 6.5, are utilized to equilibrate the anion exchange column (in this example, a capture column).

After equilibration, the loading step is performed, where feed material (post harvesting step harvested fermentation broth, of about 20 L, for example)) is loaded onto the anion exchange column at a rate of about 200 cm/hr for example. After 0.5 column volume of loaded material has passed through the anion exchange column, the flow through (FT) pool is collected into a receptacle such as a polyethersulfone vessel, while toxin complex is bound to the anion exchange column material. This is followed by a wash step, where at least about 15 column volumes of the wash buffer *(e.g.* 50 mM sodium phosphate at a pH of 6.5) is passed through the anion exchange column. The wash step is stopped when the UV, measured at the column outlet, in real time, decreases to less than or equal to about 80 mAU. The wash buffer volume and the flow through/wash pool volume are recorded, and a 1 mL sample of the flow through/wash pool is taken and tested, for example, for toxin concentration, nucleic acid content, whole cell proteins, SDS-PAGE, qPCR, 2D LC and ELISA.

The next step is the elution step, where elution buffer *(e.g.* 50 mM sodium acetate, pH 4.8) is pumped onto the anion exchange column. When the UV reading at the column outlet, in real-time, increases to about 150 mAU or more, collection of eluate in a container pre-filled with 1 CV of elution buffer (50 mM sodium acetate, pH 4.8) is begun. Collection of eluate pool is stopped when the UV reading decreases to less than or equal to about 200 mAU (volume collected at this point is between about 1 to about 2 CVs). The chromatography system is then decontaminated and cleaned using 1 N sodium hydroxide.

The eluate pool from the anion exchange column is then prepared for addition onto the cation exchange column. The anion exchange eluate volume, pH, conductivity and feed temperature are recorded and the eluate pool from the anion exchange column is diluted with 1 CV of 50 mM sodium acetate, pH 4.8.

Following the run-through of the anion exchange column, cation exchange chromatography operation is undertaken. The cation exchange column (*e.g.* Poros® 20HS) is equilibrated with a minimum of 5 CVs of equilibration buffer (50 mM sodium acetate, pH 4.8). After equilibration, the diluted eluate pool from the anion exchange column is loaded onto the cation exchange column and the total volume loaded is recorded. After 0.5 column volume of loaded diluted eluate pool has passed through the cation exchange column, the flow through (FT) pool is collected. A first wash of the cation exchange column is conducted where about 3-5 CVs of 50mM sodium acetate, pH 4.8, is passed through the cation exchange column (volume of first wash buffer utilized is recorded). A second wash is performed, where about 3 CVs of 170 mM sodium chloride, 50 mM sodium acetate, pH 4.8, is pumped through the column, this eluate being collected in a new container labeled "WASH 2 Peak". Collection is begun when the UV readings increase to greater than or equal to 50 mAU. 1 CV is collected and the second wash buffer volume utilized is recorded.

Elution of bulk toxin complex from the cation exchange column is carried out utilizing elution buffer (*e.g.* 250 mM sodium chloride in 50 mM sodium acetate, pH 4.8) which is pumped onto the cation exchange column. When the UV reading of the elution reaches at least about 100 mAU, eluate collection begins into containers pre-filled with dilution buffers (40 mL of 100 mM potassium phosphate, pH 6.8 and 60 mL of 10 mM potassium citrate, pH 6.5). Collection of eluate from the cation exchange column continues until UV readings decreases to about 100 mAU or less. The total volume of elute, after dilution, is recorded. The cation exchange chromatography system is then decontaminated and cleaned.

Following elution from the cation exchange column, the eluate is subjected to filtration. A tangential flow filtration (TFF) system is utilized, using three 100K MWCO membranes (Sartorius AG, Goettingen, Germany) stacked one atop the other. The cation exchange eluate pool initial volume is noted, as are the diafiltration/equilibration and sanitation solution descriptions. For example, the diafiltration solution can be 10 mM potassium citrate, pH 6.5 and the sanitation solution can be 0.1 N sodium hydroxide. System set up proceeds with connection of one tube from the reservoir containing either eluate from the cation column (IAPF) or HIC column (FAPF) , the eluate containing botulinum toxin, through the ultrafiltration pump head into the inlet of the tangential flow filtration membrane. A second tube from the permeate outlet of the tangential flow filtration membrane is connected to the ultrafiltration (UF) permeate container. A tube from the retentate outlet of the tangential flow filtration membrane to the retentate reservoir is secured, and a fourth tube from the diafiltration (DF) buffer through the diafiltration pump head and into the retentate reservoir is also secured. The storage buffer of the system is flushed, as is the membrane, by flushing the membrane with at least about 720 mL of water for injection (WFI) with the retentate directed to waste, after which the membrane is further flushed with at least about 4200 mL of water for injection with the retentate recirculating to the reservoir. After this, membrane sanitation (if necessary) is carried out by flushing the membrane with at least about 200 mL of 1N sodium hydroxide with the retentate directed to waste, followed by a flushing of the membrane with at least about 200 mL of 1N NaOH with the retentate recirculating to the reservoir for a minimum of 30 minutes. Equilibration is then performed, by flushing the membrane with equilibration buffer (10 mM potassium citrate at a pH of 6.5), with retentate directed to waste until the retentate and permeate pH is within +/- 0.2 units of the pH of the equilibration buffer (for example, within +/- 0.2 units of pH 6.5).

The concentration of the material (eluate (product pool) from the cation exchange column) is determined, to see if dilution or concentration (exemplary processing) is appropriate (an example target concentration can be about 0.7 mg/mL). Dilution is accomplished utilizing 10 mM potassium citrate, pH 6.5. A target volume is determined, for example for a 0.7 mg/mL product concentration (target vol= (starting concentration/starting vol)/0.7 mg/mL).

The product pool (eluate (accordingly processed or not) from cation exchange column) is loaded onto the membrane and recirculation (with permeate outlet closed) of the system (TFF system) is run for at least 2 minutes with no backpressure, after which the permeate valve is slowly opened while adjusting the retentate back pressure valve to a target of about 7 psig transmembrane pressure. For dilution, 10 mM potassium citrate, pH 6.5 is added to target volume, and moved onto diafiltration without ultrafiltration; for concentration, ultrafiltration is begun. For diafiltration: permeate waste is collected in a new container (target diafiltration volume is 5X diafiltration volume) and diafiltered with at least 5 diafiltration volumes of 10 mM potassium citrate, pH 6.5. Diafiltration process data is collected at a minimum of 10-minute intervals (permeate weight g/vol mL, inlet pressure (psig), retentate pressure (psig), permeate pressure (psig) and transmembrane pressure (psig)). For recirculation/and rinse: with the permeate outlet filter closed, the system is recirculated/run for at least 2 minutes with no backpressure and the system is rinsed with at least 20 mL of 10 mM potassium citrate, pH 6.5. The product pool includes the retentate and the rinse. A sample can be taken from the product pool and subjected to verification analysis including, for example, UV at 278nm, SDS-Page, LcHPLC, SE-HPLC, qPCR, RP-HPLC, Native-Page, AUC, Limulus amebocyte lysate, Western Blot and ELISA tests. For post-use cleaning, the system is flushed with 1N sodium hydroxide, recirculated for at least 10 minutes, after which the system is flushed and stored with 0.1 N sodium hydroxide therein.

Sterile filtration and filling are then conducted for storing and dividing the bulk neurotoxin. Concentration adjustment is performed to adjust toxin concentration, using 10 mM potassium citrate, pH 6.5, to about 0.5 mg/mL with the post rinse sample. If toxin concentration is less than about 0.5 mg/mL, then no concentration adjustment is needed.

Using a sterile pipette, 10mL/0.75mL aliquots into each of sterile 15 mL/1.5mL sample tubes are made. The product container is gently stirred by hand and transfer the required amount of solution (containing bulk drug substance, *i.e.* bulk *botulinum* toxin) into each vial. The samples are stored a maximum of 5 days at 2°C - 8°C refrigerator or 0.75 mL of

the filtrate product pool is transferred to cryovials. The cryovials are stored at -70°C +/- 5°C.

### 6.7. Example 7: A Column Chromatographic Process for Obtaining a *Botulinum* Neurotoxin

This example describes a column chromatographic process for obtaining a *botulinum* neurotoxin. It is contemplated that certain steps described in Example 5, certain steps described in Example 6, and certain steps described in this Example 7 can be combined or switched with each other for obtaining the *botulinum* neurotoxin as deemed appropriated in the opinion and judgment of a person skilled in the art.

*Clostridium botulinum bacteria* are cultured and allowed to grow until fermentation is complete. The fermentation culture is then used in the following purification procedure:

The fermentation culture is subjected to acid precipitation using 3M sulfuric acid to reduce pH to 3.5 at a temperature that is below 25°C. The acid precipitate is then subjected to 0.1 $\mu$m tangential flow filtration to concentrate cell mass. Then the pH is adjusted to 6.0 and nucleases are added to reduce host cell nucleic acid content. Then clarification by centrifugation is performed to remove cell debris and dead end filtration at 0.2 $\mu$m is performed with added ammonium sulfate. The filtrate is then directly loaded onto the hydrophobic interaction column, Phenyl Sepharose HP (GE Life Sciences), eluted with a descending gradient of ammonium sulfate, and the product peak is isolated.

Anion exchange column and/or cation exchange column are optionally used before or after the hydrophobic interaction column. If anion exchange column and/or cation exchange column are used, they are used preferably after the hydrophobic interaction column.

### 7. INCORPORATION BY REFERENCE

All references cited herein are incorporated herein by reference in their entirety and for all purposes to the same extent as if each individual publication or patent or patent application was specifically and individually indicated to be incorporated by reference in its entirety for all purposes.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

Also disclosed herein are *inter alia* the following items (said items are not claims).

1. A composition comprising a plurality of 900 kDa *Clostridium botulinum* serotype A (BoNT/A) neurotoxin complex species, wherein the oxidation level of 150 kDa neurotoxin species present in the composition is less than 6% at each of the following positions: 411 (M411) as shown in SEQ ID NO:2, 550 (M550) as show in SEQ ID NO:3, 1004 (M1004) as shown in SEQ ID NO:3, and 1144 (M1144) as shown in SEQ ID NO:3.

2. The composition of item 1, wherein the 900 kDa BoNT/A complex is onabotulinumtoxinA.

3. The composition of item 1 or 2, wherein the oxidation level at position M411 of SEQ ID NO. 2 is less than 5%.

4. The composition of any one of items 1-3, wherein the oxidation level at position M411 of SEQ ID NO. 2 is about 2%.

5. The composition of any one of items 1-4, wherein the oxidation level at position M550 of SEQ ID NO. 3 is less than 1%.

6. The composition of any one of items 1-5, wherein the oxidation level at position M550 of SEQ ID NO. 3 is about 0.3% to about 0.5%.

7. The composition of any one of items 1-6, wherein the oxidation level at position M1004 of SEQ ID NO. 3 is less than 3%.

8. The composition of any one of items 1-7, wherein the oxidation level at position M1004 of SEQ ID NO. 3 is about 1% to about 2%.

9. The composition of any one of items 1-8, wherein the oxidation level at position M1144 of SEQ ID NO. 3 is less than 6%.

10. The composition of any one of items 1-9, wherein the oxidation level at position M1144 of SEQ ID NO. 3 is about 2%.

11. The composition of any one of items 1-10, wherein the oxidation level is determined by LC-MS/MS.

12. The composition of any one of items 1-11, wherein the composition comprises less than 3% of host cell protein.

13. The composition of any one of items 1-12, wherein the composition has a potency of about $1.5 \times 10^7$ units/mg to about $6.0 \times 10^7$ units/mg.

14. The composition of item 13, wherein the potency is determined using a mouse 50% lethal dose (MLD50) assay.

15. The composition of item 13, wherein the potency is determined using a cell based potency assay.

16. The composition of any one of items 1-15 further comprising a pharmaceutically acceptable carrier.

17. The composition of item 16, wherein the pharmaceutically acceptable carrier comprises human serum albumin and sodium chloride.

18. The composition of any one of items 1-17, wherein the composition is vacuum-dried.

19. The composition of any one of items 1-18, wherein the 900 kDa BoNT/A complex is produced in a fermentation condition comprising a cold shock fermentation temperature.

20. The composition of any one of items 1-18, wherein the 900 kDa BoNT/A complex is produced in a continuous cold temperature fermentation condition.

21. The composition of any one of items 1-20, wherein the 900 kDa BoNT/A complex is purified by one or more steps of column chromatography.

22. The composition of item 21, wherein the one or more steps of column chromatography comprise hydrophobic interaction chromatography.

23. The composition of item 22, wherein the one or more steps of column chromatography further comprise anion exchange chromatography.

24. The composition of item 22 or 23, wherein the one or more steps of column chromatography further comprise cation exchange chromatography.

25. The composition of any one of items 1-24, wherein the 900 kDa BoNT/A complex is produced by *Clostridium botulinum* bacteria cultured and expanded from an animal product free working cell bank.

26. The composition of any one of items 1-25, which is animal product free.

27. The composition of any one of items 1-26, which does not contain a protease inhibitor.

28. The composition of any one of items 1-27, which does not contain benzamidine hydrochloride.

29. A method of producing a composition comprising BoNT/A, said method comprising incubating a culture of *Clostridium botulinum* bacteria in a production fermentor at a temperature that is below 35 °C for a period of time.

30. The method of item 29, wherein the entire fermentation period is about 72 hours.

31. The method of item 29, wherein the entire fermentation period is about 160 hours.

32. The method of any one of items 29-31, wherein the period of time is about 5 hours.

33. The method of any one of items 29-31, wherein the period of time is the entire fermentation period.

34. The method of any one of items 29-33, wherein the temperature is about 15 °C.

35. The method of any one of items 29-33, wherein the temperature is about 20 °C.

36. The method of any one of items 29-33, wherein the temperature is about 25 °C.

37. The method of item 29 or 30, which comprises, in the following order: (a) incubating the culture of *Clostridium botulinum* bacteria in the production fermentor at 35 °C for about 12 hours; (b) setting temperature to 15 °C at the end of step (a); (c) culturing at 15 °C until about 5 hours after step (a); (d) setting temperature to 35 °C at the end of step (c); and (e) culturing at 35 °C until about 55 hours after step (c).

38. The method of item 29 or 30, which comprises, in the following order: (a) incubating the culture of *Clostridium botulinum* bacteria in the production fermentor at 35 °C for about 12 hours; (b) setting temperature to 20 °C at the end of step (a); (c) culturing at 20 °C until about 5 hours after step (a); (d) setting temperature to 35 °C at the end of step (c); and (e) culturing at 35 °C until about 55 hours after step (c).

39. The method of item 29 or 31, which comprises incubating the culture of *Clostridium botulinum* bacteria in the production fermentor at 25 °C for about 160 hours.

40. A method of treating a patient in need thereof, comprising administering a composition according to any one of items 1-28.

41. A method substantially as described herein.

42. A composition substantially as described herein.

**Claims**

1. A method of producing a 900 kDa BoNT/A complex, wherein the method comprises culturing a *Clostridial botulinum* bacteria, and purifying the 900 kDa BoNT/A complex by one or more steps of column chromatography, wherein the purification does not comprise precipitation with cold ethanol, hydrochloric acid, or ammonia sulfate.

2. A method for preparing a *Clostridium botulinum* working cell bank (WCB), the method comprising:

   (i) obtaining a research cell bank (RCB) from a *Clostridium botulinum* master cell bank (MCB);
   (ii) expanding cells from the RCB in an animal product free (APF) liquid medium under anaerobic conditions to obtain an expanded culture;
   (iii) adding glycerol to the expanded culture to form a glycerol containing cell suspension; and
   (iv) dispensing the glycerol containing cell suspension into cryovials and flash freezing the vials in liquid nitrogen to obtain the WCB.

*FIG. 1*

EP 4 768 580 A2

*FIG. 2*

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63480938
- US 20030118598
- US 8618261 B
- US 8198034 B
- US 9249216 B
- US 10703806 B
- US 11261240 B
- US 11332518 B
- US 7354740 B
- US 8129139 B
- US 7160699 B
- US 9725705 B
- US 9920310 B
- WO 2010105234 A
- WO 2009114748 A
- DE 60555131
- US 7452697 B

### Non-patent literature cited in the description

- **ZHANG et al.** *Gene*, 2003, vol. 315, 21
- **LIETZOW et al.** *Protein J*, 2008, vol. 27, 420-425
- **HUNT et al.** *Toxins*, 2010, vol. 2 (8), 2198-2212
- **RUPP et al.** *Toxins*, 2020, vol. 12 (6), 393
- **SCHANTZ** ; **KAUTTER**. *Journal of the AOAC*, 1978, vol. 61 (1), 96-99
- **HUNT** ; **KENNETH**. *Clinical Neuropharmacology*, 2009, vol. 32 (1), 28-31
- **AOKI et al.** *Eur. J. Neurol.*, 1999, vol. 6, s3-s10
- **AOKI**. *Toxicon*, 2001, vol. 39, 1815-1820
- **BROIDE et al.** *Toxicon*, 2013, vol. 71, 18-24
- **EKONG et al.** *Microbiology*, 1997, vol. 143, 3337-3347
- **FERNÁNDEZ-SALAS et al.** *PLOS ONE*, 2012, vol. 7, 11
- **FERNANDEZ-SALAS et al.** *PLOS ONE*, 2012, vol. 7 (11), e49516
- **BENOIT et al.** *Sci Rep*, 2017, vol. 7, 43588
- **BENOIT et al.** *Sci Rep.*, 2017, vol. 7, 43588
- **FERNÁNDEZ-SALAS et al.** *PLOS ONE*, 2012, vol. 7 (11), e49516